Europäisches Patentamt

European Patent Office

Office européen des brevets

(19)

(11) Publication number: **0 258 046**
A2

## (12) EUROPEAN PATENT APPLICATION

(21) Application number: 87307538.6

(22) Date of filing: 26.08.87

(51) Int. Cl.⁴: **C 12 N 15/00**
C 12 N 1/20
//(C12N1/20,C12R1:465)

(30) Priority: 28.08.86 US 901240   28.08.86 US 901334

(43) Date of publication of application:
02.03.88   Bulletin 88/09

(84) Designated Contracting States:
BE CH DE FR GB IT LI NL SE

(71) Applicant: ELI LILLY AND COMPANY
Lilly Corporate Center
Indianapolis Indiana 46285 (US)

(72) Inventor: Epp, Janet Kay
920 North Graham Avenue
Indianapolis Indiana 46219 (US)

Schoner, Brigitte Elisabeth
Rural Route No. 2 Box 30F
Monrovia Indiana 46157 (US)

(74) Representative: Hudson, Christopher Mark et al
Erl Wood Manor
Windlesham Surrey GU20 6PH (GB)

(54) Method for selecting recombinant DNA-containing streptomyces and other host cells.

(57) The present invention provides a method for selecting a recombinant DNA-containing Streptomyces host cell that comprises transforming the host cell with a carbomycin resistance-conferring vector and culturing the transformants under conditions suitable for selection of carbomycin-resistant transformants. The invention also relates to DNA compounds, genes, and vectors useful in the method as well as transformants selected by the method.

EP 0 258 046 A2

**Description**

METHOD FOR SELECTING RECOMBINANT DNA-CONTAINING STREPTOMYCES AND OTHER HOST CELLS

The present invention relates to a method for selecting a recombinant DNA-containing host cell, particularly a Streptomyces host cell, that involves selecting for the carbomycin-resistant phenotype. The invention also relates to novel carbomycin resistance-conferring genes, designated carA and carB, recombinant DNA cloning vectors that comprise the novel genes, and transformants prepared by the method that contain the carbomycin resistance-conferring vectors. Streptomyces thermotolerans (ATCC 11416) produces carbomycin, a macrolide antibiotic consisting of a 16-member cyclic lactone and two sugar residues. The antibiotic activity of carbomycin, like that of other macrolides, is due to inhibition of protein synthesis by a mechanism that involves the binding of carbomycin to the ribosome.

The present invention provides a method for selecting host cells transformed with carbomycin resistance-conferring cloning vectors. The method is particularly useful in Streptomyces and other prokaryotic host cells. The development and exploitation of recombinant DNA technology in Streptomyces and other organisms depends upon the availability of methods for identifying the transformed cells, which, in turn, depends upon the availability of selectable genetic markers on suitable cloning vectors. This development has been somewhat retarded by the low number of selectable markers presently available for use in Streptomyces. The present invention is useful and especially important in that it provides a novel method for selecting recombinant DNA-containing Streptomyces and expands the number of selectable. markers suitable for use in recombinant DNA technology.

The vectors used in the present method are particularly useful, because the vectors are small, versatile, and can be transformed and selected in a variety of carbomycin-sensitive Streptomyces cells. Streptomyces provides over half of the clinically . important antibiotics and thus is a commercially significant group. The present invention relates to new and useful cloning systems and vectors for this industrially important group and allows for the cloning of genes both for increasing the yields of known antibictics and also for producing new antibiotics and antibiotic derivatives.

The present invention further relates to vectors that enable identification of Streptomyces transformants. After the addition of non-selectable DNA to a vector useful in the method of the present invention, the modified vector can be transformed into Streptomyces and transformants identified by their carbomycin-resistant phenotype. Because transformation is a relatively low frequency event, such a functional test is a practical necessity for determining which cell(s), of among the millions of cells, has acquired the transforming DNA.

For purposes of the present invention, as disclosed and claimed herein, the following terms are defined below.

ApR - the ampicillin-resistant phenotype or gene conferring same.

carA - a carbomycin resistance-conferring gene of type A.

carB - a carbomycin resistance-conferring gene of type B.

mel - the tyrosinase gene.

Phasmid--a recombinant DNA vector that may act as a phage or as a plasmid.

Recombinant DNA Cloning Vector--any autonomously replicating or integrating agent, including, but not limited to, plasmids, comprising a DNA molecule to which one or more additional DNA segments can be or have been added.

Restriction Fragment--any linear DNA molecule generated by the action of one or more restriction enzymes.

Sensitive Host Cell--a host cell that cannot grow in the presence of a given antibiotic without a DNA segment that provides resistance thereto.

TcR - the tetracycline-resistant phenotype or gene conferring same.

Transformant--a recipient host cell that has undergone transformation.

Transformation--the introduction of DNA into a recipient host cell that changes the genotype and results in a change in the recipient cell.

tsrR - the thiostrepton-resistant phenotype or gene conferring same.

The figures described below are drawn to scale; however, observed restriction fragment size may vary somewhat from calculated size based on map distances. For some restriction enzymes, such as MboI, only the significant cut sites are shown.

Figure 1 is a restriction site and function map of plasmid pOJ158.

Figure 2 is a restriction site and function map of plasmid pOJ159.

Figure 3 is a restriction site and function map of plasmid pOJ160.

Figure 4 is a restriction site and function map of plasmid pOJ169.

Figure 5 is a restriction site and function map of plasmid pOJ197.

The present invention relates to a method for selecting a recombinant DNA-containing host cell that comprises transforming a carbomycin-sensitive host cell with a recombinant DNA vector that codes for expression of a carbomycin resistance-conferring gene product and culturing the transformed cell under conditions suitable for selection of carbomycin-resistant transformants. The invention also relates to novel DNA compounds, genes, and vectors useful in the method together with carbomycinresistant transformants obtained by using the method. Two novel and distinct genes, designated carA and carB, are particularly useful and preferred for use in the present method.

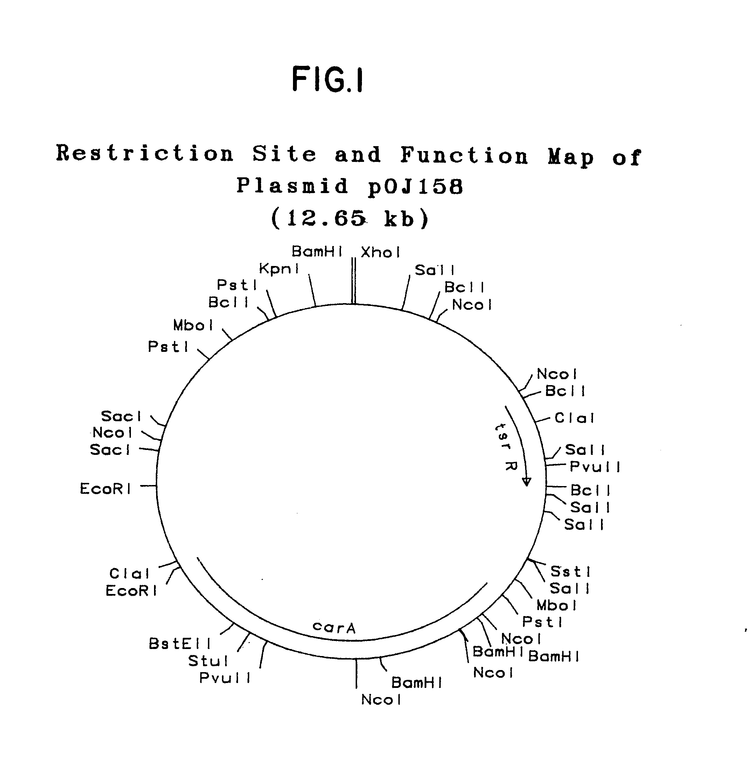

The carA gene can be isolated from plasmid pOJ158 on an 4 kb PstI-EcoRI restriction fragment; plasmid pOJ158 can be isolated from Streptomyces griseofuscus C581/pOJ158, a strain deposited and made part of the permanent culture collection of the Agricultural Research Service, Northern Regional Research Center (NRRL), Peoria, IL 61604, under the accession number NRRL 18089. A restriction site and function map of plasmid pOJ158 is presented in Figure 1 of the accompanying drawings. Plasmid pOJ158 can be isolated from S. griseofuscus C581/pOJ158 in substantial accordance with the procedure described in Example 1.

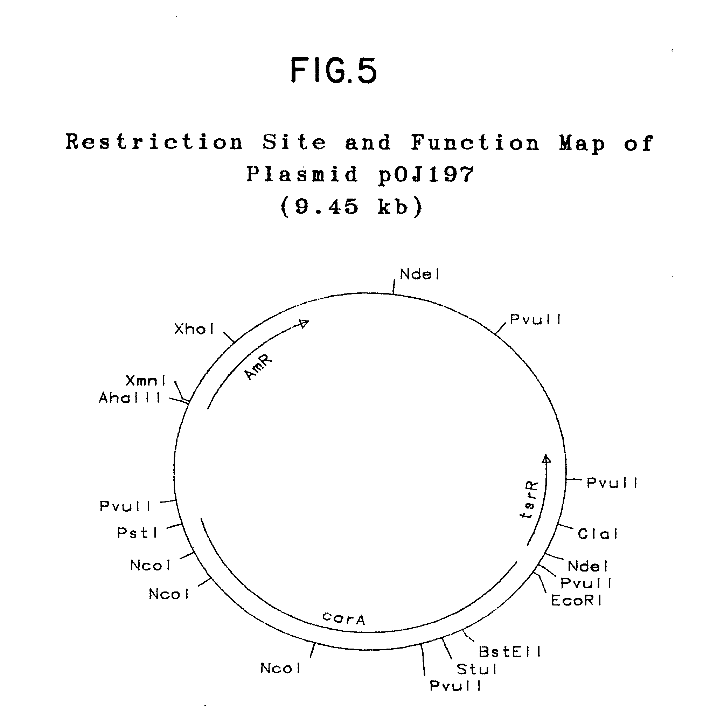

Plasmid pOJ158 serves as useful starting material for the construction of other vectors that confer carA-mediated carbomycin resistance. For example, the 4 kb PstI-EcoRI, carbomycin resistance-conferring restriction fragment of plasmid pOJ158 was isolated and inserted into EcoRI-PstI-digested plasmid pOJ160 (NRRL B-18088) to yield plasmid pOJ197. The construction protocol for plasmid pOJ197 is given in Example 2; a restriction site and function map of plasmid pOJ197 is presented in Figure 5 of the accompanying drawings.

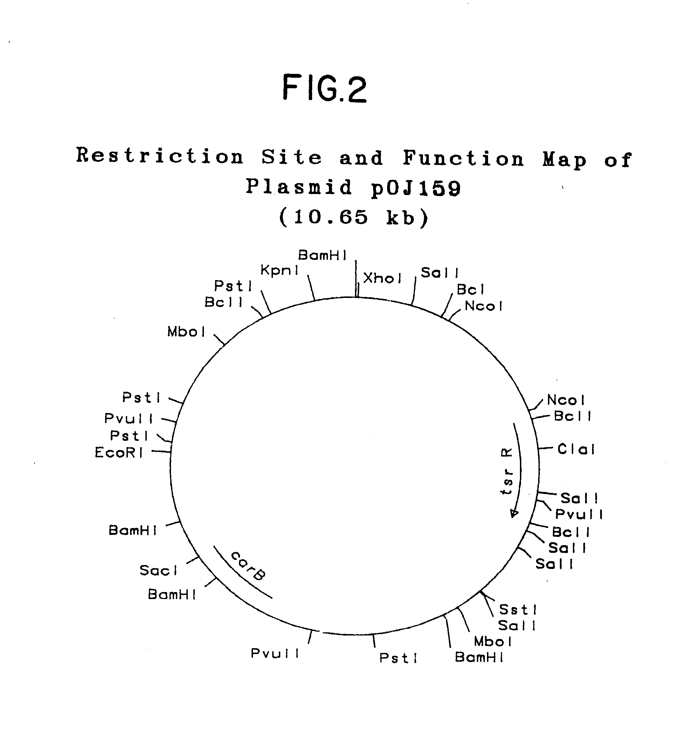

The carB gene can be isolated from plasmid pOJ159 on an 3.0 kb PstI restriction fragment; plasmid pOJ159 can be isolated from Streptomyces griseofuscus C581/pOJ159, a strain deposited and made part of the permanent culture collection of the Agricultural Research Service, Northern Regional Research Center (NRRL), Peoria, IL 61604, under the accession number NRRL 18090. A restriction site and function map of plasmid pOJ159 is presented in Figure 2 of the accompanying drawings. Plasmid pOJ159 can be isolated from S. griseofuscus C581/pOJ159 in substantial accordance with the procedure described in Example 4.

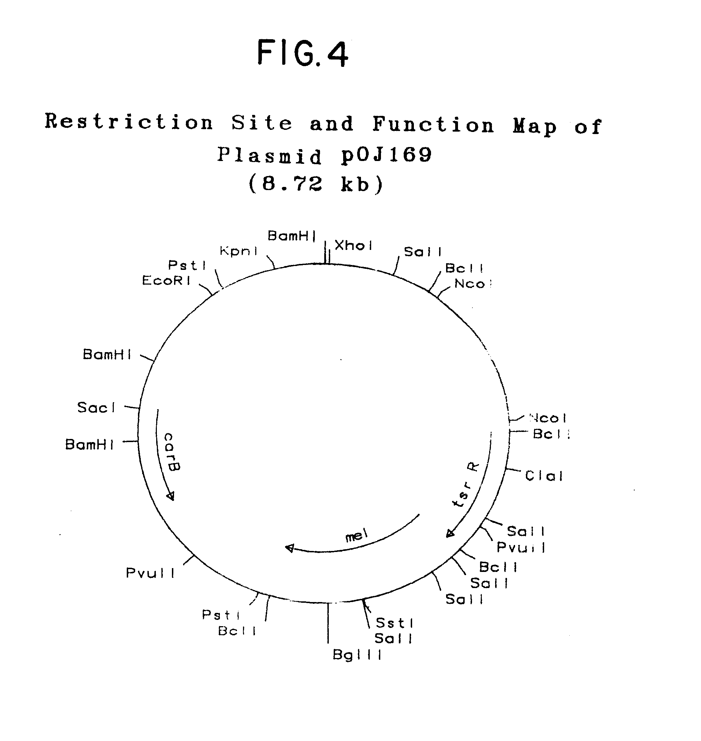

Plasmid pOJ159 serves as useful starting material for the construction of other vectors that confer carB-mediated carbomycin resistance. For example, the 3.0 kb PstI, carbomycin resistance-conferring restriction fragment of plasmid pOJ159 was isolated and inserted into PstI-digested plasmid pIJ702 (ATCC 39155) to yield plasmids pOJ169 and pOJ170, which differ only with respect to the orientation of the 3.0 kb PstI, carbomycin resistance-conferring restriction fragment. The construction protocol for plasmids pOJ169 and pOJ170 is given in Example 5; a restriction site and function map of plasmid pOJ169 is presented in Figure 4 of the accompanying drawings.

The carA and carB genes were isolated from a carbomycin-producing strain of Streptomyces thermotolerans (ATCC 11416). Thus, genomic DNA of S. thermotolerans was partially digested with restriction enzyme MboI, and the resulting DNA was inserted into BglII-digested plasmid pIJ702 to yield a number of carA-containing and carB-containing plasmids, including plasmids pOJ158 and pOJ159. Because the carA and carB genes were isolated from S. thermotolerans, the carA and carB genes function in S. thermotolerans, but the two genes with their natural promoter also function in other organisms.

The vectors of the present invention have also been used to transform Streptomyces lividans TK23 (NRRL 15826) and Streptomyces griseofuscus C581 (ATCC 23916) to carbomycin resistance, as described in Examples 3 and 6. Thus, the carA and carB genes can be used to transform a variety of carbomycin-sensitive Streptomyces strains to carbomycin resistance. In organisms naturally sensitive to macrolide antibiotics, including carbomycin, the carA and carB genes can be used as a genetic marker. In organisms that produce one or more macrolide antibiotics yet are sensitive to low levels of macrolide antibiotic, the vectors of the present invention can be used to increase or augment the organism's natural resistance. The following Tables present a representative sampling of various antibiotic-producing organisms in which the carA and carB genes can be used.

## TABLE I

### Aminocyclitol Antibiotic-Producing Organisms

| Organism | Antibiotic |
|---|---|
| Bacillus | |
| various species | various aminocyclitols |
| Micromonospora | |
| various species | gentamycins |
| Saccharopolyspora | |
| various species | various aminocyclitols |
| Streptomyces | |
| albogriseolus | neomycins |
| albus var. metamycinus | metamycin |
| aquacanus | N-methyl hygromycin B |
| atrofaciens | hygromycins |
| bikiniensis | streptomycin |
| bluensis var. bluensis | bluensomycin |
| canus | ribosyl paromamine |
| catenulae | catenulin |
| chrestomyceticus | aminosidine |
| crystallinus | hygromycin A |
| erythrochromogenes | |
| var. narutoensis | streptomycin |
| eurocidicus | A16316-C |
| fradiae | hybrimycins and neomycins |
| fradiae var. italicus | aminosidine |
| galbus | streptomycin |
| griseus | streptomycin |

## TABLE I (Continued)

Aminocyclitol Antibiotic-Producing Organisms

| Organism | Antibiotic |
|---|---|
| Streptomyces | |
| griseoflavus | MA 1267 |
| hofuensis | seldomycin complex |
| hygroscopicus | hygromycins, leucanicidin, and hygrolidin |
| hygroscopicus forma glebosus | glebomycin |
| hygroscopicus var. limoneus | validamycins |
| hygroscopicus var. sagamiensis | spectinomycin |
| kanamyceticus | kanamycin A and B |
| kasugaensis | kasugamycins |
| kasugaspinus | kasugamycins |
| lavendulae | neomycin |
| lividus | lividomycins |
| mashuensis | streptomycin |
| microsporeus | SF-767 |
| netropsis | LL-AM31 |
| noboritoensis | hygromycins |
| olivaceus | streptomycin |
| olivoreticuli var. cellulophilus | destomycin A |
| poolensis | streptomycin |
| rameus | streptomycin |
| ribosidificus | SF733 |
| rimofaciens | destomycin A |
| rimosus forma paromomycinus | paromomycins and catenulin |
| spectabilis | spectinomycin |
| tenebrarius | tobramycin and apramycin |
| Streptoverticillium | |
| flavopersicus | spectinomycin |

## TABLE II

### Ansamycin Antibiotic-Producing Organisms

| Organism | Antibiotic |
|---|---|
| <u>Micromonospora</u> | |
| various species | various ansamycins |
| <u>Nocardia</u> | |
| <u>mediterranei</u> | rifamycin |
| <u>Streptomyces</u> | |
| <u>collinus</u> | ansatrienes and napthomycins |
| <u>diastochromogenes</u> | ansatrienes and napthomycins |
| <u>galbus</u> subsp. <u>griseosporeus</u> | napthomycin B |
| <u>hygroscopicus</u> | herbimycin |
| <u>hygroscopicus</u> var. <u>geldanus</u> var. <u>nova</u> | geldamycin |
| <u>nigellus</u> | 21-hydroxy-25-demethyl 25-methylthioproto- streptovaricin |
| <u>rishiriensis</u> | mycotrienes |
| sp. E/784 | actamycin and mycotrienes |
| sp. E88 | mycotrienes |
| <u>spectabilis</u> | streptovaricins |
| <u>tolypophorous</u> | tolypomycin |

6

## TABLE III

Anthracycline and Quinone Antibiotic-Producing Organisms

| Organism | Antibiotic |
|---|---|
| Streptomyces | |
|     caespitosus | mitomycins A, B, and C |
|     coelicolor | actinorhodin |
|     coeruleorubidicus | daunomycin |
|     cyaneus | ditrisarubicin |
|     flavogriseus | cyanocycline A |
|     galilaeus | aclacinomycin A, auramycins, and sulfurmycins |
|     lusitanus | napthyridinomycin |
|     peuceticus | daunomycin and adriamycin |
|     violochromogenes | arugomycin |

## TABLE IV

β-Lactam Antibiotic-Producing Organisms

| Organism | Antibiotic |
|---|---|
| Agrobacterium | various β-lactams |
| Cephalosporium | |
|     acremonium | penicillins and cephalosporins |
| Chromobacterium | various β-lactams |
| Gluconobacter | various β-lactams |
| Nocardia | |
|     lactamadurans | cephamycin C |
|     uniformis | nocardicin |
| Penicillium | |
|     chrysogenum | various penicillins and other β-lactams |
| Serratia | various β-lactams |

## TABLE IV (Continued)

### β-Lactam Antibiotic-Producing Organisms

| Organism | Antibiotic |
|---|---|
| Streptomyces | |
| antibioticus | clavulanic acid |
| argenteolus | asparenomycin A, MM 4550, and MM 13902 |
| cattleya | thienamycin |
| chartreusis | SF 1623 and cephamycin A and B |
| cinnamonensis | cephamycin A and B |
| clavuligerus | PA-32413-I, cephamycin C, A16886A, penicillins cephalosporins, clavulanic acid, and other clavams |
| fimbriatus | cephamycin A and B |
| flavovirens | MM 4550 and MM 13902 |
| flavus | MM 4550 and MM 13902 |
| fulvoviridis | MM 4550 and MM 13902 |
| griseus | cephamycin A and B and carpetimycin A and B |
| halstedi | cephamycin A and B |
| heteromorphus | C2081X and cephamycin A and B |
| hygroscopicus | deacetoxycephalosporin C |
| lipmanii | cephamycin, penicillin N, 7-methoxycephalosporin C, A16884, MM4550, MM13902 |
| olivaceus | epithienamycin F, MM 4550, and MM 13902 |
| panayensis | C2081X and cephamycin A and B |
| rochei | cephamycin A and B |
| sioyaensis | MM 4550 and MM 13902 |
| sp. OA-6129 | OA-6129A |
| sp. KC-6643 | carpetimycin A |
| viridochromogenes | cephamycin A and B |
| wadayamensis | WS-3442-D |

TABLE V

Macrolide, Lincosamide, and Streptogramin
Antibiotic-Producing Organisms

| Organism | Antibiotic |
|---|---|
| Micromonospora | |
| rosaria | rosaramicin |
| Streptomyces | |
| albireticuli | carbomycin |
| albogriseolus | mikonomycin |
| albus . | albomycetin |
| albus var. | |
| coilmyceticus | coleimycin |
| ambofaciens | spiramycin and foromacidin D |
| antibioticus | oleandomycin |
| avermitilis | avermectins |
| bikiniensis | chalcomycin |
| bruneogriseus | albocycline |
| caelestis | M188 and celesticetin |
| cinerochromogenes | cineromycin B |
| cirratus | cirramycin |
| deltae | deltamycins |
| djakartensis | niddamycin |
| erythreus | erythromycins |
| eurocidicus | methymycin |
| eurythermus | angolamycin |
| fasciculus | amaromycin |
| felleus | argomycin and picromycin |
| fimbriatus | amaromycin |
| flavochromogenes | amaromycin and shincomycins |
| fradiae | tylosin |
| fungicidicus | NA-181 |
| fungicidicus var. | |
| espinomyceticus | espinomycins |
| furdicidicus | mydecamycin |
| goshikiensis | bandamycin |
| griseofaciens | PA133A and B |
| griseoflavus | acumycin |
| griseofuscus | bundlin |
| griseolus | griseomycin |
| griseospiralis | relomycin |
| griseus | borrelidin |

9

## TABLE V (Continued)

Macrolide, Lincosamide, and Streptogramin
Antibiotic-Producing Organisms

| Organism | Antibiotic |
|---|---|
| Streptomyces | |
| griseus ssp. sulphurus | bafilomycins |
| halstedi | carbomycin and leucanicidin |
| hygroscopicus | tylosin |
| hygroscopicus subsp. aureolacrimosus | milbemycins |
| kitastoensis | leucomycin $A_3$ and josamycin |
| lavendulae | aldgamycin |
| lincolnensis | lincomycin |
| loidensis | vernamycin A and B |
| macrosporeus | carbomycin |
| maizeus | ingramycin |
| mycarofaciens | acetyl-leukomycin, and espinomycin |
| narbonensis | josamycin and narbomycin |
| narbonensis var. josamyceticus | leucomycin $A_3$ and josamycin |
| olivochromogenes | oleandomycin |
| platensis | platenomycin |
| rimosus | tylosin and neutramycin |
| rochei | lankacidin and borrelidin |
| rochei var. volubilis | T2636 |
| roseochromogenes | albocycline |
| roseocitreus | albocycline |
| spinichromogenes var. suragaoensis | kujimycins |
| tendae | carbomycin |
| thermotolerans | carbomycin |
| venezuelae | methymycins |
| violaceoniger | lankacidins and lankamycin |

**0 258 046**

<u>TABLE VI</u>

Miscellaneous Antibiotic-Producing <u>Streptomyces</u>

| <u>Antibiotic Type</u> | <u>Streptomyces</u> Species | <u>Antibiotic</u> |
|---|---|---|
| amino acid analogues | sp. | cycloserine |
| cyclopentane ring-containing | <u>coelicolor</u><br><u>erythrochromogenes</u><br><u>Kasugaensis</u><br><br><u>violaceoruber</u> | methylenomycin A<br>sarkomycin<br>aureothricin and<br>thiolutin<br>methylenomycin A |
| nitro-containing | <u>venezuelae</u> | chloramphenicol |
| polyenes | <u>griseus</u><br><u>nodosus</u><br><u>noursei</u> | candicidin<br>amphotericin B<br>nystatin |
| tetracyclines | <u>aureofaciens</u><br><br><br><br><br><br><u>rimosus</u> | tetracycline, chlor-tetracycline, demethyltetra-cycline, and demethylchlortetra-cycline<br>oxytetracycline |

11

## TABLE VII

### Nucleoside Antibiotic-Producing Organisms

| Organism | Antibiotic |
|---|---|
| Corynebacterium michiganese pv. rathayi | tunicamycin analogues |
| Nocardia candidus | pyrazofurin |
| Streptomyces antibioticus | ara-A |
| chartreusis | tunicamycin |
| griseoflavus var. thuringiensis | streptoviridans |
| griseolus | sinefungin |
| lysosuperificus | tunicamycin |

## TABLE VIII

### Peptide Antibiotic-Producing Organisms

| Organism | Antibiotic |
|---|---|
| Actinoplanes missouriensis | actaplanin |
| teichomyceticus | teicoplanin |
| Bacillus various species | bacitracin, polymixin, and colistin |
| Nocardia candidus | A-35512 and avoparcin |
| lurida | ristocetin |
| orientalis | vancomycin |
| Streptomyces antibioticus | actinomycin |
| aureus | thiostrepton |
| canus | amphomycin |
| eburosporeus | LL-AM374 |
| haranomachiensis | vancomycin |
| pristinaespiralis | pristinamycin |
| roseosporus | lipopeptides, such as A21978C |
| toyocaensis | A47934 |
| virginiae | A41030 |

12

## TABLE IX

Polyether Antibiotic-Producing Organism

| Organism | Antibiotic |
|---|---|
| Actinomadura | |
| various species | various polyethers |
| oligosporus | A80190 |
| | |
| Dactylosporangium | |
| various species | various polyethers |
| | |
| Nocardia | |
| various species | various polyethers |
| | |
| Streptomyces | |
| albus | A204, A28695A and B, and salinomycin |
| aureofaciens | narasin |
| bobili | A80438 |
| cacaoi var. | |
| asoensis | lysocellin |
| chartreusis | A23187 |
| cinnamonensis | monensin |
| conglobatus | ionomycin |
| eurocidicus var. | |
| asterocidicus | laidlomycin |
| flaveolus | CP38936 |
| gallinarius | RP 30504 |
| griseus | grisorixin |
| hygroscopicus | A218, emericid, DE3936, A120A, A28695A and B, etheromycin, and dianemycin |
| lasaliensis | lasalocid |
| longwoodensis | lysocellin |
| mutabilis | S-11743a |
| pactum | A80438 |
| ribosidificus | lonomycin |
| violaceoniger | nigericin |
| | |
| Streptoverticillium | |
| various species | various polyethers |

As both the carA and carB genes were isolated from Streptomyces thermotolerans, the carA and carB genes should function in most Streptomyces species. Yet even if the intact carA or carB gene failed to express

13

in a given organism, such as E. coli, because, for example, the Streptomyces promoter failed to function in that organism, the carA or carB protein-coding sequence of the present invention could be ligated to a DNA containing an appropriate promoter and ribosome-binding site to achieve expression of the respective carA or carB gene product. Thus, both the carA protein-coding sequence and the carB protein-coding sequence are useful in all carbomycin-sensitive, host cells.

Plasmid pOJ158 contains the complete carA gene: (1) a promoter that directs transcription of the protein-coding sequence; (2) a sequence that, when transcribed into mRNA, directs translation of the transcript; (3) a protein-coding sequence and (4) a transcription terminator. Each of these elements is independently useful and can, through the techniques of recombinant DNA technology, be used to form recombinant genes of great variety. The DNA sequence of the carA gene can be obtained using standard techniques (Maxam and Gilbert, 1980, Methods in Enzymology 65:449-560 and Sanger et al., Proc. Natl. Acad. Sci. 74:5463-5467) and will reveal the location of the carA coding sequence. Such information allows one to position other promoters in reading phase with the carA coding sequence. By choosing the proper promoter, one can construct vectors that drive expression of the carA gene product in any given host cell.

The promoter of the carA gene is useful in its own right. The promoter and other regulatory elements of the carA gene can be linked to the coding sequence of a non-carbomycin antibiotic biosynthetic gene to prepare a hybrid antibiotic pathway gene that functions in another Streptomyces species to yield a hybrid antibiotic. Thus, the individual elements of the carA gene on the plasmids described herein comprise important components of the present invention.

Those skilled in the art will recognize that the carA sequence deposited under accession number NRRL 18089 can be used to prepare synthetic or natural DNA probes for use in obtaining other macrolide, lincosamide, and streptogramin (MLS) resistance-conferring genes. In addition, due to the diversity of Streptomyces thermotolerans strains both in nature and also in the laboratory, there will be a variety of allelic variants of the carA gene that can be readily isolated by using the carA gene as a probe to identify homologous DNA. These allelic variants, which encode gene products with an amino acid residue sequence that differs from that of the carA gene product in only a few residues, are equivalent to the carA gene of the present invention.

The DNA sequence past, or downstream of, the termination codon of the carA coding sequence comprises a transcription terminator. Transcription terminators can impart stability upon transcripts and play a regulatory role. Restriction fragments containing the transcription termination sequence of the carA gene can be conveniently isolated from Streptomyces thermotolerans DNA and used to prepare recombinant genes for expression in Streptomyces.

Plasmid pOJ159 contains the complete carB gene: (1) a promoter that directs transcription of the protein-coding sequence; (2) a sequence that, when transcribed into mRNA, directs translation of the transcript; (3) a protein-coding sequence; and (4) a transcription terminator. Each of these elements is independently useful and can, through the techniques of recombinant DNA technology, be used to form recombinant genes of great variety. The DNA sequence of the carB gene, depicted below, reveals the location of the carB coding sequence and thus allows one to position other promoters, for example, the trp, lpp, and lac promoters of E. coli and the veg promoter of Bacillus, in reading phase with the carB coding sequence. By choosing the proper promoter, one can construct vectors that drive expression of the carB gene product in any given host cell.

The promoter of the carB gene is useful in its own right. The promoter and other regulatory elements of the carB gene can be linked to the coding sequence of a non-carbomycin antibiotic biosynthetic gene to prepare a hybrid antibiotic pathway gene that functions in another Streptomyces species to yield a hybrid antibiotic. Thus, the individual elements of the gene on the plasmids described herein comprise important components of the present invention.

The sequence of the carB gene was obtained by first cloning the 3 kb carbomycin resistance-conferring PstI restriction fragment of plasmid pOJ159 into PstIdigested plasmid SP64 to yield plasmids pOJ168 and pOJ168.1, which differ only with respect to the orientation of the 3 kb PstI restriction fragment. Plasmid SP64 is a "riboprobe" vector marketed by Promega Biotec, 2800 S. Fish Hatchery Road, Madison, Wisconsin 53711. The pertinent region of plasmid pOJ168 was then sequenced (Maxam and Gilbert, 1980, Methods in Enzymology 65:449-560 and Sanger et al., 1977, Proc. Natl. Acad. Sci. 74:5463-5467) to determine the nucleotide sequence of the carB gene. The sequence of the carB gene is depicted below, beginning with the 5′ end located upstream of the coding sequence. Only the sequence of the coding strand of the carB gene is depicted; the sequence of the non-coding strand can be obtained using the well-known rules of base-paring: A pairs with T, and C pairs with G. The amino acid residue sequence of the carB gene product is also depicted below, beginning with the amino-terminal end; each amino acid residue is located below the DNA encoding that residue. Both the DNA and amino acid residue sequences are numbered to facilitate discussion.

NUCLEOTIDE SEQUENCE OF THE carB GENE AND AMINO ACID RESIDUE
SEQUENCE OF THE carB GENE PRODUCT


```
              10         20          30         40
     5'-CGCTGGACTG CCGCCCGGTT TTGAGCAGGC ATTTCGCCCG

              50         60          70         80
        TCCGCCCGCA CGGCGGGGCC AGCCACGGGC CGGTGTCCGC

              90        100         110        120
        CGCCGGCGCC GCGCCACCCC CACGGCCGCA TCCCGTACCC

             130        140         150        160
        GGGCTGCGGC CGCTTCGGGT TGTCCTCTGC CGCGGCTTGA

             170        180         190        200
        TTTCACCTCC CCGGGGGGTG TGCGTAGTGG CAGCGAAGGT

             210        220         230        240
        AAAGGGAGGT TAAGGTGTCC GTCCGCCGGG GTTCCTGTAC

             250        260         270        280
        GTAATGGCTG CGCTCCTGAA GCGCATACTT AGGAGACGCA

        290        300         310          320           330
TGGCTGAAAA GAGGTCAGGA CGCGGGCGC ATG GCC GCA GCG CGT ACA ACC GGA GCT
                                   MET ALA ALA ALA ARG THR THR GLY ALA
                                                              - 5
      340         350         360          370           380
CAG TCG CGT AAA ACG GCA CAG CGG TCG GGC CGG AGT GAG GCT GAC CGT
GLN SER ARG LYS THR ALA GLN ARG SER GLY ARG SER GLU ALA ASP ARG
10                  15                  20                  25
      390         400         410          420           430
AGA AGA AGA GTC CAC GGG CAG AAT TTC CTC GTC GAC CGG GAA ACA GTA
ARG ARG ARG VAL HIS GLY GLN ASN PHE LEU VAL ASP ARG GLU THR VAL
                30              35                  40
      440         450         460          470           480
CAA CGG TTT GTG CGT TTC GCC GAT CCG GAC CCC GGG GAG GTC GTT CTC
GLN ARG PHE VAL ARG PHE ALA ASP PRO ASP PRO GLY GLU VAL VAL LEU
         45                  50                  55
```

```
                490              500              510              520
GAG GTC GGT GCC GGT AAT GGT GCG ATC ACG CGC GAG CTG GCG CGA TTA
LEU GLU VAL GLY ALA GLY ASN GLY ALA ILE THR ARG GLU LEU ALA ARG
        60               65               70
 530         540              550         560         570
TGC CGA CGA GTG GTG GCG TAT GAG ATC GAC CGG CAC TTC GCG GAC CGA
CYS ARG ARG VAL VAL ALA TYR GLU ILE ASP ARG HIS PHE ALA ASP ARG
        75               80               85
        580         590              600         610         620
TTA CGT GAG GCG ACC GCC GAG GAT CCG CGG ATC GAG GTC GTC GCC GGC
LEU ARG GLU ALA THR ALA GLU ASP PRO ARG ILE GLU VAL VAL ALA GLY
90               95              100                        105
         630         640         650         660         670
GAC TTC CTG AAG ACC TCG CAG CCC AAG GTC CCG TTC TCC GTG GTC GGC
ASP PHE LEU LYS THR SER GLN PRO LYS VAL PRO PHE SER VAL VAL GLY
             110                      115              120
             680         690         700         710         720
AAC ATC CCG TTC GGC AAC ACC GCG GAC ATA GTG GAC TGG TGC CTG AAC
ASN ILE PRO PHE GLY ASN THR ALA ASP ILE VAL ASP TRP CYS LEU ASN
             125                      130              135
             730         740         750         760
GCG CGG CGG CTG CGT ACG ACC ACC CTG GTC ACC CAG CTC GAA TAC GCC
ALA ARG ARG LEU ARG THR THR THR LEU VAL THR GLN LEU GLU TYR ALA
         140                      145              150
 770         780         790         800         810
CGC AAG CGC ACC GGC GGC TAT CGG CGC TGG TCA CGG CTC ACC GTG GCC
ARG LYS ARG THR GLY GLY TYR ARG ARG TRP SER ARG LEU THR VAL ALA
     155             160                  165
     820         830         840         850         860
ACC TGG CCC GAG GTG GAG TGG CGG ATG GGC GAG CGG ATC AGC CGC CGC
THR TRP PRO GLU VAL GLU TRP ARG MET GLY GLU ARG ILE SER ARG ARG
170                      175              180                  185
     870         880         890         900         910
TGG TTC CGG CCC GTC CCC GCC GTC GAC TCC GCG GTA CTG CGA CTG GAA
TRP PHE ARG PRO VAL PRO ALA VAL ASP SER ALA VAL LEU ARG LEU GLU
             190                      195              200
         920         930         940         950         960
CGG CGA CCG GTG CCG CTG ATC CCA CCC GGT CTG ATG CAC GAC TTC CGG
ARG ARG PRO VAL PRO LEU ILE PRO PRO GLY LEU MET HIS ASP PHE ARG
         205                      210              215
             970         980         990         1000
GAC CTG GTG GAG ACC GGG TTC ACG GGA AAG GGC GGT TCG CTG GAC GCC
ASP LEU VAL GLU THR GLY PHE THR GLY LYS GLY GLY SER LEU ASP ALA
         220                      225              230
```

```
     1010          1020          1030          1040          1050
TCG CTG CGC CGG CGC TTC CCG GCC CGG CGG GTG GCC GCC GGG TTC CGC
SER LEU ARG ARG ARG PHE PRO ALA ARG ARG VAL ALA ALA GLY PHE ARG
    235                     240                     245
          1060          1070          1080          1090          1100
AGG GCC CGC CTG GAG CAG GGC GTG GTC GTC GCC TAC GTC ACC CCG GGC
ARG ALA ARG LEU GLU GLN GLY VAL VAL VAL ALA TYR VAL THR PRO GLY
250                     255                     260                     265
         1110          1120          1130          1140          1150
CAA TGG ATC ACA CTC TTC GAG GAA CTC CAC GGG CGC TGA CGCACAC
GLN TRP ILE THR LEU PHE GLU GLU LEU HIS GLY ARG
                   270                     275
               1160          1170          1180          1190          1200
      CGGTGCGGGG CGCGCCCGAG GGCGCGCCCC GCACCGGTCG TCCCGCGTCG


               1210          1220          1230          1240          1250
      GCTACGGAGT GCCGGACGGG GCGGGTTCGG ACCTGGGTGT CGAGCCGGCG


               1260
      TCCGGGGACC-3'
```

wherein A is deoxyadenyl, G is deoxyguanyl, C is deoxycytidyl, T is thymidyl, ALA is an alanine, ARG is an arginine, ASN is an asparagine, ASP is an aspartic acid, CYS is a cysteine, GLN is a glutamine, GLU is a glutamic acid, GLY is a glycine, HIS is a histidine, ILE is an isoleucine, LEU is a leucine, LYS is a lysine, MET is a methionine, PHE is a phenylalanine, PRO is a proline, SER is a serine, THR is a threonine, TRP is a tryptophan, TYR is a tyrosine, and VAL is a valine.

There is some uncertainty as to the precise location of the beginning of the carB coding region, because there are three in-frame methionine codons (ATG) located within the first 100 bp of the open-reading frame at positions 244, 280 and 310. Translation from these sites would generate proteins with 299, 287 and 277 amino acids, respectively. Only the third of the three ATG codons (at position 310) is preceeded by an appropriately spaced Shine-Dalgarno (SD) 5'-AGGA-3' with a window size of 9 nucleotides. A second SD sequence (GAGG) occurs 13 nucleotides upstream from this ATG codon, but a window size of 13 bp places this SD sequence further upstream than has been generally observed. Similarly, a SD sequence (GGAG), located 4 nucleotides upstream from the ATG codon at position 280 is closer than what has been generally observed. No SD sequence is associated with the ATG codon at position 244. Although a GTG codon could be utilized for translation initiation, this seems less likely as the first GTG codon in the sequence appears at position 442 and has no associated SD sequence. Although not wishing to be bound by theory, we believe translation of the carB open reading frame begins at position 310; therefore, we have depicted the start of the amino acid residue sequence of carB at position 310.

Those skilled in the art will recognize that the carB sequence depicted above and deposited under accession number NRRL 18090 can be used to prepare synthetic or natural DNA probes for use in obtaining other RNA methylase-encoding DNA segments and macrolide, lincosamide, and streptogramin (MLS) resistance-conferring genes. In addition, due to the diversity of Streptomyces thermotolerans strains both in nature and also in the laboratory, there will be a variety of allelic variants of the carB gene that can be readily isolated given the sequence of the carB gene depicted above. These allelic variants, which encode gene products with an amino acid residue sequence that differs from that of the carB gene product in only a few residues, are equivalent to the carB gene of the present invention.

The DNA sequence past the termination codon (TGA), at position 1141, contains a GC-rich inverted repeat that potentially can form a very stable hairpin-loop structure, herein defined as a transcription terminator. The stem in this structure, which roughly comprises the sequence from position 1141 to position 1194, contains 18 perfectly matched base pairs and a calculated ΔG of -70 kcals. These structures conventionally represent transcription terminators and can impart stability upon the transcripts and play a regulatory role. The structure of the hairpin-loop structure is depicted below.

```
            AG
         C     G
          C-G
          C-G
        · G-C
          C-G
          G-C
          C-G
          G-C
          G-C
          G-C
          G-C
          C-G
          G-C
          T-A
          G-C
          G-C
          C-G
          C-G
 5'-TGACGCACA-TCGTCCC-3'
```

Restriction fragments containing the termination sequence depicted above can be conveniently isolated from plasmid pOJ159, or can be synthesized using manual or automated techniques, and used to prepare recombinant genes for expression in Streptomyces.

The method of the present invention is not limited to a particular carbomycin resistance-conferring gene or vector. Thus, even though many of the vectors described above comprise the Streptomyces replicon derived from plasmid pIJ702, a variety of known Streptomyces replicons can be used to construct equally useful vectors, with respect to the present method, with different host ranges. Table X is an illustrative, but not comprehensive, listing of Streptomyces plasmids from which Streptomyces replicons can be obtained. Those skilled in the art recognize that, so long as the replicon function is not disrupted, all or part of the plasmids can be used to construct vectors that contain the carB gene of the present invention. The plasmid-containing host and depository accession number are also listed in Table X.

## Table X

### Streptomyces Plasmids

| Plasmid | Host | Accession Number |
|---|---|---|
| SCP2 | Streptomyces coelicolor A3(2) | NRRL 15042 |
| SCP2* | Streptomyces coelicolor M110 | NRRL 15041 |
| pEL7 | Streptomyces ambofaciens/pEL7 | NRRL 12523 |
| pUC6 | Streptomyces espinosus | NRRL 11439 |
| pUC3 | Streptomyces 3022A | NRRL 11441 |
| SLP1 | Streptomyces lividans | NCIB* 11417 |
| pNM100 | Streptomyces virginiae | NRRL 15156 |
| pEL103 | Streptomyces granuloruber A399 12.13/pEL103 | NRRL 12549 |
| pIJ702 | Streptomyces lividans | ATCC** 39155 |

*National Collection of Industrial Bacteria (NCIB), Torry Research Station, Post Office Box 31, 135 Abbey Road, Aberdeen AB98DG, Scotland, United Kingdom.

**American Type Culture Collection, Rockville, MD 20852.

Restriction fragments used to construct vectors illustrative of the present invention can be conventionally modified to facilitate ligation. For example, molecular linkers can be provided to a particular carbomycin resistance gene-containing restriction fragment or to DNA comprising vector replication or integration functions. Thus, specific sites for subsequent ligation can be conveniently constructed. In addition, the various carbomycin resistance gene- containing restriction fragments, origin of replication, or sequences that provide for chromosomal integration of a given vector can be modified by adding, eliminating, or substituting certain nucleotides to alter characteristics and to provide a variety of restriction sites for ligation of DNA. Those skilled in the art understand nucleotide chemistry and the genetic code and thus which nucleotides are interchangeable and which DNA modifications are desirable for a specific purpose. It is also noteworthy that a given carbomycin resistance gene-containing restriction fragment is not limited to a particular position on a cloning vector, as long as critical, vector-controlled functions are not disrupted. Those skilled in the art understand or can readily determine which sites on a vector are advantageous for the ligation or insertion of a particular carbomycin resistance gene-containing restriction fragment.

The present method for selecting recombinant DNA-containing host cells by transforming the host cell with a vector that drives expression of a carbomycin resistance-conferring gene product and culturing the transformed host cell under conditions suitable for selection of carbomycin-resistant transformants is not limited to a particular type of expression vector. The vector can be a plasmid, phage or other type of vector, so long as the vector confers resistance to carbomycin.

For instance, either the carA or the carB gene can be used to construct vectors other than plasmids. Phage φC31 is a well-known Streptomyces phage that is an excellent source of starting material for constructing integrative carbomycin resistance-conferring vectors that further exemplify the present invention. A derivative of phage φC31, plasmid pKC331, is especially preferred for constructing such integrating vectors and can be obtained from E. coli K12 BE447/pKC331 (NRRL B-15828). φC31-type phages are integrative vectors and can be readily modified to incorporate either the carA or the carB gene, or both, and thus confer carbomycin resistance to Streptomyces.

The vectors of the present invention comprise a Streptomyces replicon and a carbomycin resistance-conferring restriction fragment. Because amplification and manipulation of plasmids is done faster and more efficiently in E. coli than in Streptomyces, it is convenient to add DNA sequences that also allow for replication in E. coli. Thus, the addition of functional replicon-containing and antibiotic resistance-conferring restriction fragments from E. coli plasmids such as, for example, pBR322, pACYC184, pBR325, pBR328, and the like is highly advantageous and adds to the general utility of the present illustrative vectors.

The carA gene confers resistance to carbomycin and other antibiotics. In Streptomyces griseofuscus, the carA confers resistance to carbomycin, spiramycin, and lincomycin. In S. lividans, carA increases resistance to erythromycin and oleanodomycin. Inducible resistance to macrolide antibiotics, such as carbomycin and erythromycin, in Gram-positive bacteria (such as Staphylococcus, Streptomyces, Streptococcus, and Bacillus) is associated with co-resistance to lincosamide and streptogramintype B antibiotics; this multi-drug-resistant phenotype is called the MLS-resistant phenotype (Fujisawa and Weisblum, 1981, J. Bacteriol. 146:621-631).

The carA gene confers a modified MLS-resistant phenotype. Table XI, below, presents the results of disc-sensitivity assays, which demonstrate that the carA gene confers resistance to a number of antibiotics. The carA-containing cells were cultured in 0.1 μg/ml carbomycin prior to the assays. The carA gene can confer resistance to a greater variety of MLS antibiotics when cells containing the carA gene are cultured under inducing conditions prior to selection with antibiotic, i.e., induction with erythromycin or oleandomycin in S. griseofuscus cells containing the carA gene makes the cells resistant to tylosin. Inducing conditions are achieved by exposing the cells to subinhibitory concentrations of certain macrolide antibiotics and other antibiotics that bind to the 50S subunit of the ribosomes. Different species of Streptomyces will exhibit different induction patterns.

## Table XI

## Antibiotic Resistance Pattern

| Antibiotic[a] | S. thermotolerans | S. griseofuscus | |
|---|---|---|---|
| | | pIJ702 | pOJ158 |
| erythromycin | 23 | R[b] | R |
| oleandomycin | 15 | R | R |
| carbomycin | R | 26 | R |
| spiramycin | R | 20 | R |
| tylosin | 16 | 24 | 17 |
| rosamycin | R[b] | 37 | 15 |
| lincomycin | R | 13 | R[b] |
| vernamycin B | R[b] | 16 | 11[c] |

[a] 20 µg of antibiotic per disc; numbers refer to zone diameters; R indicates no zone.

[b] small zone of growth inhibition.

[c] turbid zone.

The relatively large zones of inhibition of growth around the discs show that Streptomyces griseofuscus/pIJ702 is quite sensitive to MLS antibiotics, except for erythromycin and oleandomycin. In the presence of plasmid pOJ158, the zone sizes are considerably smaller or absent, because of increased resistance to these antibiotics as a result of expression of the carA gene. Vernamycin B gave a turbid zone that is due to inducible or partial resistance to vernamycin B. The small, but clearly discernable zones around the lincomycin disc shows that this antibiotic causes inhibition of growth at lower concentrations than do the other antibiotics tested. The minimal concentration of carbomycin that inhibits growth is given in Table XII, below. Resistance to erythromycin and oleandomycin was analyzed in S. lividans, because this strain is sensitive to these antibiotics. S. lividans transformants containing the carA gene are more resistant to erythromycin and oleandomycin even though S. thermotolerans is sensitive to these antibiotics.

The information in Tables XI and XII illustrates that the level of macrolide antibiotic required to observe growth inhibition of a sensitive strain varies and depends on the particular antibiotic, host strain, and growth conditions. Those skilled in the art will recognize that the precise experimental conditions, including the preferred macrolide antibiotic concentration, for using the carA gene for purposes of selection can be readily determined by empirical methods. A preferred procedure for determining an organism's sensitivity to carbomycin utilizes antibiotic-gradient plates, as exemplified in Table XII, to measure the minimum inhibitory concentration (MIC) of antibiotic needed to inhibit cell growth. Disc-sensitivity assays can also be used to determine an organism's sensitivity to carbomycin, as exemplified in Table XI. The vectors of the present invention can increase the endogenous resistance to carbomycin by about 100 fold. Variations in this increase are expected when different promoters are used to drive expression of the carA gene.

## Table XII

| Strain | MIC (μg/ml) carbomycin[a] |
|---|---|
| S. griseofuscus | 0.5 |
| S. griseofuscus/pIJ702 | 0.5 |
| S. griseofuscus/pOJ158 | 30 |
| S. thermotolerans | >1000 |

[a] values were determined on antibiotic gradient plates (Szybalski, 1952, Science 116:46-48)

The carB gene confers resistance to carbomycin and other antibiotics. In Streptomyces griseofuscus, carB confers resistance to carbomycin, spiramycin, rosamycin, lincomycin, and vernamycin B. In S. lividans, carB additionally confers resistance to erythromycin and oleandomycin. Inducible resistance to macrolide antibiotics, such as tylosin, carbomycin, and erythromycin, in Gram-positive bacteria (such as Staphylococcus, Streptomyces, Streptococcus, and Bacillus) is associated with co-resistance to lincosamide and streptogramin-type B antibiotics; this multi-drug-resistant phenotype is called the MLS-resistant phenotype (Fujisawa and Weisblum, 1981, J. Bacteriol. 146:621-631). In Staphylococcus aureus, the MLS-resistant phenotype arises upon specific methylation of an adenine residue in 23S ribosomal RNA (rRNA) resulting in the formation of $N^6$-dimethyladenine. MLS-resistance in Streptomyces erythreus, an erythromycin producer, arises upon $N^6$-dimethylation of a single adenine residue in 23S rRNA. Thompson et al. cloned a S. erythreus gene, ermE, that conferred erythromycin resistance in S. lividans via a mechanism that generated $N^6$-dimethyladenine in 23S rRNA.

The carB gene may encode a rRNA methylase, because of the extensive amino acid sequence homology between the RNA methylase encoded by the ermE gene and the protein encoded by carB. However, the carB gene confers a modified MLS-resistant phenotype, and Table XIII, below, presents the results of disc-sensitivity assays, which demonstrate that the carB gene confers resistance to a number of antibiotics. The results presented in Table XIII are based on uninduced culture conditions. The carB gene can confer resistance to a greater variety of MLS antibiotics, such as tylosin, when cells containing the carB gene are cultured under inducing conditions prior to selection with antibiotic. Inducing conditions are achieved by exposing the cells to sub-inhibitory concentrations of certain macrolide anti biotics and other antibiotics that bind to the 50S subunit of the ribosomes. Different species of Streptomyces will exhibit different induction patterns.

Table XIII

Antibiotic Resistance Pattern

| Antibiotic[a] | S. thermotolerans | S. griseofuscus pIJ702 | S. griseofuscus pOJ159 | S. lividans pIJ702 | S. lividans pOJ159 |
|---|---|---|---|---|---|
| erythromycin | 23 | R[b] | R | 20 | R[b,c] |
| oleandomycin | 15 | R | R | 15 | R |
| carbomycin | R | 26 | R[b] | 15(20[c]) | R |
| spiramycin | R | 20 | R | 12 | R |
| tylosin | 16 | 24 | 18 | 16 | 11[c] |
| rosamycin | R[b] | 37 | 11[c] | 11[c] | R |
| lincomycin | R | 13 | R | 12 | R |
| vernamycin B | R[b] | 16 | R[b] | 11[c] | R[b,c] |

[a] 20 µg of antibiotic per disc; numbers refer to zone diameters; R indicates no zone.

[b] small zone of growth inhibition.

[c] turbid zone.

The relatively large zones of inhibition of growth around the discs show that Streptomyces griseofuscus is quite sensitive to MLS antibiotics, except for erythromycin and oleandomycin. In the presence of plasmid pOJ159, the zone sizes are considerably smaller or absent, because of increased resistance to these antibiotics as a result of expression of the carB gene. Rosamycin gave a turbid zone that is due to inducible or partial resistance to rosamycin. The small, but clearly discernable zones around vernamycin and carbomycin show that these antibiotics cause inhibition of growth at lower concentrations than do the other antibiotics tested. The minimal concentration of carbomycin that inhibits growth is given in Table XIV, below. Resistance to erythromycin and oleandomycin was analysed in S. lividans because this strain is sensitive to these antibiotics. The results depicted in Table XIV demonstrate the S. lividans transformants are resistant to erythromycin and oleandomycin even though S. thermotolerans is sensitive to these antibiotics.

The information in Tables XIII and XIV indicates that the level of macrolide antibiotic required to observe growth inhibition of a sensitive strain varies and depends on the particular antibiotic, host strain, and growth conditions. Those skilled in the art will recognize that the precise experimental conditions, including the preferred macrolide antibiotic concentration, for using the carB gene for purposes of selection can be readily determined by empirical methods. A preferred procedure for determining an organism's sensitivity to carbomycin utilizes antibiotic-gradient plates, as exemplified in Table XIV. Disc-sensitivity assays can also be used to determine an organism's sensitivity to carbomycin, as exemplified in Table XIII. The carB-containing vectors of the present invention can increase the endogenous resistance to carbomycin by about 100 fold. Variations in this increase are expected when different promoters are used to drive expression of the carB gene.

## Table XIV

| Strain | MIC ($\mu$g/ml) carbomycin[a] |
|---|---|
| S. griseofuscus | 0.5 |
| S. griseofuscus/pIJ702 | 0.5 |
| S. griseofuscus/pOJ159 | 50 |
| S. thermotolerans | >1000 |

[a]values were determined on antibiotic gradient plates (Szybalski, 1952, Science 116:46-48)

Streptomyces thermotolerans contains two carbomycin resistance-conferring genes, designated carA and carB. The level of carbomycin resistance in the S. griseofuscus host cells transformed with the vectors of the invention was lower than in S. thermotolerans, because the two carbomycin resistance-conferring genes act in concert to cause high-level resistance in the wild-type antibiotic-producing organism. Thus, there may be advantages in using a vector comprising both the carA and the carB genes in the method of the present invention.

The recombinant DNA cloning vectors of the present invention have broad utility and help fill the need for suitable cloning vehicles for use in Streptomyces and related organisms. Moreover, the ability of the present vectors to confer carbomycin resistance provides a functional means for selecting transformants. This is important because of the practical necessity for determining and selecting the particular cells that have acquired vector DNA in a transformation procedure.

Additional DNA segments, that lack functional tests for their presence, can also be inserted into the present vectors, and transformants containing the non-selectable DNA can be isolated by selection for carbomycin resistance. Such non-selectable DNA segments can be inserted at any site, except within regions. necessary for plasmid function and replication or within the particular carbomycin resistance-conferring gene used on the vector, and include, but are not limited to, genes that specify antibiotic modification enzymes and regulatory genes of all types.

More particularly, a non-selectable DNA segment that comprises a gene is inserted into a plasmid such as, for example, plasmid pOJ169 at the central ClaI restriction site of the thiostrepton resistance gene. Such an insertion inactivates the thiostrepton resistance gene and thus allows for the easy identification of transformants containing the recombinant plasmid. This is done by first selecting for carbomycin resistance and, secondarily, identifying those carbomycin-resistant transformants that are not resistant to thiostrepton. Therefore, the ability to select for carbomycin resistance in Streptomyces and related cells allows for the efficient isolation of the relatively few cells that contain the particular non-selectable DNA of interest.

The functional test for carbomycin resistance, described above, is also used to locate DNA segments that act as control elements and direct expression of an individual antibiotic resistance-conferring gene. Such segments, including, but not limited to, promoters, attenuators, repressors, inducers, ribosome-binding sites, and the like, are used to control the expression of other genes in Streptomyces and related organisms.

The carbomycin resistance-conferring vectors of the present invention are also useful for ensuring that linked DNA segments are stably maintained in host cells over many generations. These genes or DNA fragments, covalently linked to the carbomycin resistance-conferring DNA and propagated in Streptomyces, are maintained by exposing the transformants to levels of carbomycin toxic to non-transformed cells. Therefore, transformants that lose the vector, and consequently lose any covalently linked DNA, cannot grow and are eliminated from the culture. Thus, the vectors of the present invention can stabilize and maintain DNA sequences of interest.

The cloning vectors and transformants of the present invention provide for the cloning of genes to improve yields of various products that are currently produced in Streptomyces and related cells. Examples of such products include, but are not limited to, Streptomycin, Tylosin, Cephalosporins, Actaplanin, Narasin, Monensin, Tobramycin, Erythromycin, and the like. The present invention also provides selectable vectors that are useful for cloning, characterizing, and reconstructing DNA sequences that code for: commercially important proteins such as, for example, human insulin, human proinsulin, glucagon, interferon and the like; enzymatic functions in metabolic pathways leading to commercially important processes and compounds; or control elements that improve gene expression. These desired DNA sequences also include, but are not limited to, DNA that codes for enzymes that catalyze synthesis of derivatized antibiotics such as, for example, Streptomycin, Cephalosporin, Tylosin, Actaplanin, Narasin, Monensin and Erythromycin derivatives, or for enzymes that mediate and increase bioproduction of antibiotics or other products. The capability for isolating and using such DNA segments allows for increasing the yield and availability of antibiotics that are produced by Streptomyces and related organisms.

Streptomyces can be cultured in a number of ways using any of several different media. Preferred carbohydrate sources in a culture medium include, for example, molasses, glucose, dextrin, and glycerol. Nitrogen sources include, for example, soy flour, amino acid mixtures, and peptones. Nutrient inorganic salts are also incorporated and include the customary salts capable of yielding sodium, potassium, ammonium, calcium, phosphate, chloride, sulfate, and like ions. As is necessary for the growth and development of other micro-organisms, essential trace elements are also added. Such trace elements are commonly supplied as impurities incidental to the addition of other constituents of the medium.

Streptomyces is grown under aerobic culture conditions over a relatively wide pH range of about 5 to 9 at temperatures ranging from about 15° to 40°C. For plasmid stability and maintenance, it is desirable to start with a culture medium at a pH of about 7.2 and maintain a culture temperature of about 30°C.

The following examples further illustrate and describe the invention disclosed herein. The invention is not limited in scope by reason of any of the following Examples; sources of reagents or equipment are provided merely for convenience and in no way limit the invention. Both an explanation of and the actual procedures for constructing the invention are described where appropriate.

Example 1

Isolation of Plasmid pOJ158

A. Culture of Streptomyces griseofuscus C581/pOJ158

About $10^8$ spores of Streptomyces griseofuscus C581/pOJ158 (NRRL 18089) are inoculated into 10 ml of TSB medium (Trypticase Soy Broth, TSB, is made at 30 g/l and obtained from Baltimore Biological Laboratories (BBL), P.O. Box 243, Cockeysville, Maryland (21031) containing 25 µg/ml thiostrepton and grown at 29°C until the culture is in early stationary phase. The culture was then homogenized, and 5 ml of the homogenized culture were used to inoculate 100 ml of TSB also containing thiostrepton. The 100 ml of culture were incubated at 29°C until the Streptomyces griseofuscus C581/pOJ158 cells reached stationary phase.

B. Plasmid Isolation

The cells were collected and washed once with a 10.3% sucrose solution. The cells were then suspended in 24 ml of 10.3% sucrose, and 6 ml of 5X lysozyme solution (125 mM Tris-HCl, pH = 8; 125 mM Na$_2$EDTA, pH = 8; 10 mg/ml lysozyme; and 10.3% sucrose) were added. The solution was mixed and then incubated at 30°C for 30-60 minutes, and then, about 18 ml of a solution that was 0.3 M NaOH, 1% SDS, and prewarmed to 50°C were added, mixed and the resulting mixture incubated at 80°C for 10 minutes. The mixture was then cooled to room temperature, and 12 ml of a solution made by mixing 500 g phenol and 500 g CHCl$_3$ in 200 ml H$_2$O were added and mixed well with the cell-extract. The phases were separated by centrifugation at 6000-8000 rpm for 10 minutes; approximately 45 ml of the resulting upper phase were transferred to a clean bottle.

Next, 4.5 ml of 3 M sodium acetate (NaOAc) and 50 ml of isopropanol were added to the supernatant, and the solution was mixed and left at room temperature for 30 minutes. The solution was then centrifuged (8000 rpm for 30 minutes) and the resulting supernatant discarded. The pellet was resuspended in 10 ml of TE buffer (10 mM Tris-HCl, pH = 8, and 1 mM EDTA) containing 9.5 g of CsCl. About 1 ml of a 5 mg/ml solution of ethidium bromide was added to the solution to bring the final volume to 12.5 ml. The solution was then centrifuged at 52,000 rpm for 48 hours at 20°C in a Beckman Ti-75 fixed-angle rotor. The fraction containing the plasmid band was extracted 5 times with isopropanol saturated with 20X SSC (0.3 M NaCl and 0.3 M NaCitrate) to remove the ethidium bromide. After the extractions, the sample was dialyzed against 1000 volumes of $H_2O$ and then against 1500 volumes of TE buffer. The procedure yields about 100 μg of plasmid pOJ158 DNA at a concentration of 0.2 μg/μl, which is stored at 4°C. A restriction site and function map of plasmid pOJ158 is presented in Figure 1 of the accompanying drawings.

Example 2

Construction of Plasmid pOJ197

A. Isolation of Plasmid pOJ160

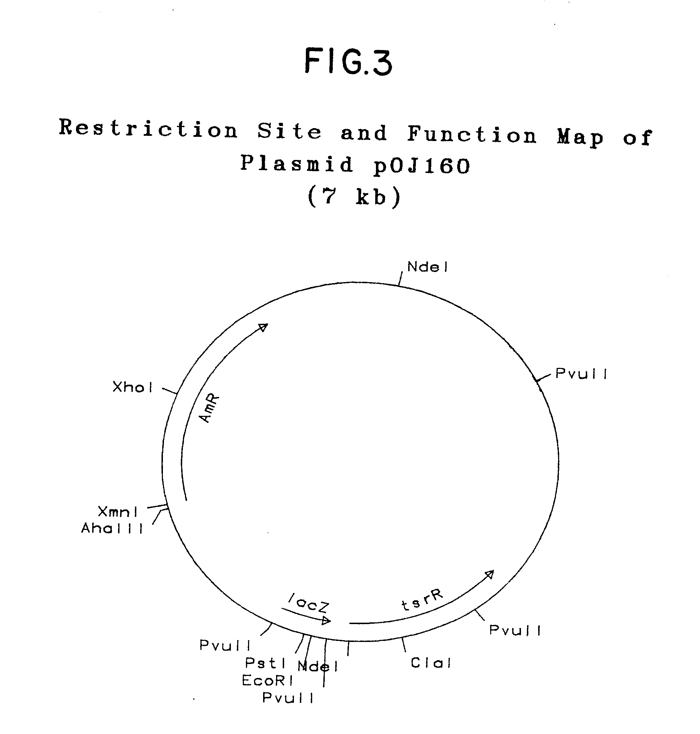

Plasmid pOJ160 (Figure 3) can be obtained from the Northern Regional Research Center in E. coli K12 JM109 under the accession number NRRL B-18088. The lyophils of E. coli K12 JM109/pOJ160 are plated onto L-agar plates (10 g of Bacto-tryptone, 10 g of NaCl, 5 g of Bacto-Yeast Extract, and 15 g of agar per liter) containing 200 μg/ml apramycin to obtain a single colony isolate of the strain. This colony is used to inoculate about 500 ml of L broth (L agar without agar) containing 200 μg/ml apramycin, and the resulting culture is incubated at 37°C with aeration until the cells reach stationary phase.

Plasmid DNA was obtained from the cells to use in construction of plasmid pOJ197 in accordance with the following procedure, which is adapted from Maniatis et al., 1982, Molecular Cloning (Cold Spring Harbor Laboratory). This same procedure was used, but on a smaller scale and with the ultracentrifugation steps replaced with phenol followed by chloroform extractions, to prepare the plasmid DNA used to identify the E. coli K12 RR1ΔM15/pOJ197 transformants.

About 500 ml of stationary-phase E. coli/pOJ160 cells are harvested by centrifugation at 4000Xg for 10 minutes at 4°C, and the supernatant is discarded. The cell pellet is washed in 100 ml of ice-cold STE buffer (0.1 M NaCl; 10 mM Tris-HCl, pH 7.8; and 1 mM EDTA). After the cell pellet is washed, the pellet is resuspended in 10 ml of Solution 1 (50 mM glucose; 25 mM Tris-HCl, pH=8.0; and 10 mM EDTA) that contains 5 mg/ml lysozyme and is left at room temperature for 10 minutes. Twenty ml of Solution 2 (0.2 N NaOH and 1% SDS) are then added to the lysozyme-treated cells, and the solution is gently mixed by inversion. The mixture is incubated on ice for 10 minutes.

Fifteen ml of ice-cold, 3 M sodium acetate, pH=4.8, are added to the lysed-cell mixture, and the solution is mixed by inversion. The solution is incubated on ice for 60 minutes. The 3 M sodium acetate solution is prepared by mixing equal volumes of 3 M acetic acid and 3 M sodium acetate.

The lysed cell mixture is centrifuged in a Beckman SW27 rotor (or its equivalent) at 20,000 rpm for 20 minutes at 4°C. About 36 ml of supernatant are recovered, and 2.5 volumes of ethanol are added, mixed, and the resulting solution left on ice for 15 minutes. The plasmid DNA is collected by centrifugation at 12,000Xg for 30 minutes at room temperature. The supernatant is discarded, and the DNA pellet is washed with 70% ethanol at room temperature. The ethanol wash is decanted, and the pellet is dried in a vacuum desiccator. The pellet is then resuspended in 8 ml of TE buffer.

Eight grams of CsCl are added to the DNA solution. About 0.8 ml of a 10 mg/ml solution of ethidium bromide in water are added for each 10 ml of CsCl-DNA solution. The final density of the solution is about 1.55 g/ml, and the ethidium bromide concentraton is about 800 μg/ml. The solution is transferred to a Beckman Type 50 centrifuge tube, filled to the top with TE buffer containing 1.55 g/ml CsCl, sealed, and centrifuged at 45,000 rpm for 24 hours at 20°C. After centrifugation, two bands of DNA are visible in ordinary light and become even more prominent in UV light. The cap is removed from the tube, and the lower DNA band is recovered using a syringe with a #21 hypodermic needle inserted through the side of the centrifuge tube.

The ethidium bromide is removed from the solution of plasmid DNA by several extractions with water-saturated 1-butanol, and the CsCl is removed by dialysis against TE buffer. After extractions with buffered phenol and then chloroform, the DNA is pre cipitated, washed with 70% ethanol, and dried. About 0.5 mg of plasmid pOJ160 DNA can be obtained by this procedure.

B. Final Construction of Plasmid pOJ197

About 1 μg (1 μl) of plasmid pOJ160 DNA was added to 2 μl of 10X PstI buffer (500 mM Tris-HCl, pH=7.5; 0.5 M NaCl; and 100 mM MgCl₂), 16 μl of $H_2O$, and 1 μl (15 units; unit definitions herein correspond to those of New England Biolabs, 32 Tozer Road, Beverly, MA 01915-9990, unless otherwise indicated) of restriction enzyme PstI. The resulting reaction was incubated at 37°C for two hours. The reaction mixture was extracted with 100 μl of a 1:1 solution of phenol:chloroform and then with 100 pl of chloroform. The PstI-digested DNA was collected by adjusting the sodium acetate (NaOAc) concentration of the reaction mixture to 0.30 M, adding 2.5 volumes of ethanol, chilling the reaction mixture to -70°C, and centrifuging to pellet the precipitated DNA. The pellet of PstI-digested plasmid pOJ160 DNA was resuspended in 1 μl of 10X EcoRI buffer (1.0 M Tris-HCl, pH=7.5; 0.5 M NaCl; 50 mM MgCl₂; and 1 mg/ml BSA), 18 μl of $H_2O$, and 1 μl (20 units) of restriction enzyme EcoRI. The resulting reaction was incubated at 37°C for 2 hours; then, the reaction mixture was extracted and the DNA collected as described above. The EcoRI-PstI-digested plasmid pOJ160 DNA was resuspended in 1 μl of $H_2O$.

About 10 μg of plasmid pOJ158 in 100 μl of TE buffer were added to 13 μl of 10X PstI buffer, 13 μl of H₂O and 4 μl (60 units) of restriction enzyme PstI. The resulting reaction was incubated at 37°C for 2 hours. The reaction mixture was extracted and the DNA collected as described above; then, the PstI-digested plasmid pOJ158 DNA was resuspended in 10 μl of 10X EcoRI buffer, 3 μl (60 units) of restriction enzyme EcoRI, and 87 μl of H₂O. The reaction was incubated at 37°C for 2 hours.

The PstI-EcoRI-digested plasmid pOJ158 DNA was then electrophoresed on a 1% agarose gel until the desired 4 kb PstI-EcoRI restriction fragment was clearly separated from the other digestion products. Visualization of the electrophoresed DNA was accomplished by staining the gel in a dilute solution (0.5 μg/ml) of ethidium bromide and exposing the stained gel to longwave UV light. After the desired fragment was located, a small slit was made in the gel in front of the fragment, and a small piece of Schleicher and Schuell (Keene, NH 03431) NA-45 DEAE membrane was placed in the slit. Upon further electrophoresis, the 4 kb PstI-EcoRI restriction fragment was non-covalently bound to the DEAE membrane. After the desired fragment was bound to the DEAE membrane, the membrane was removed and rinsed with low salt buffer (150 mM NaCl; 0.1 mM EDTA; and 20 mM Tris-HCl, pH = 8). Next, the membrane was placed in a small tube and immersed in high salt buffer (1 M NaCl; 0.1 mM EDTA; and 20 mM Tris-HCl, pH = 8) and then incubated at 65°C for one hour to elute the DNA from the DEAE paper. After the 65°C incubation, the incubation buffer was collected and the membrane rinsed with high salt buffer. The rinse solution was pooled with the incubation buffer before collecting the desired DNA fragments.

About three volumes of cold, absolute ethanol were added to the high salt-DNA solution. The resulting solution was mixed and placed on ice for 10-20 minutes and then centrifuged at 15,000 rpm for 15 minutes. After another precipitation to remove residual salt, the DNA pellet was rinsed with ethanol, dried, resuspended in 50 μl of TE buffer, and constituted 2.5 pg of the desired 4 kb PstI-EcoRI restriction fragment of plasmid pOJ158.

The PstI-EcoRI-digested plasmid pOJ160 DNA (1 μl) was added to 10 μl (0.5 μg) of the 4 kb, carA-containing PstI-EcoRI restriction fragment of plasmid pOJ158, 2 μl of 10X ligase buffer (660 mM Tris-HCl, pH = 8; 66 mM MgCl₂; 10 mM dithiothreitol (DTT); and 10 mM ATP), and 6 μl of H₂O. About 1 μl (100 units) of T4 DNA ligase was added to the solution of DNA, and the resulting reaction was incubated at 15°C overnight (16 hours). The ligated DNA constituted the desired plasmid pOJ197; a restriction site and function map of plasmid pOJ197 is presented in Figure 5 of the accompanying drawings.

The EcoRI and PstI sites on plasmid pOJ160 reside within a polylinker that itself forms part of the DNA sequence encoding the lacZ α-fragment. Expression of the lacZ α-fragment in an E. coli ΔM15 strain, such as E. coli Kl2 RR1ΔM15 (NRRL B-15440), restores the strain's ability to produce a functional β-galactosidase enzyme. Thus, plasmid pOJ160 can restore β-galactosidase activity to the E. coli K12 RR1ΔM15 strain. However, insertion of DNA into a restriction site of the polylinker on plasmid pOJ160, as occurs in the construction of plasmid pOJ197, disrupts the lacZ α-fragment coding sequence and concomitantly destroys the ability of the plasmid pOJ160 derivative to complement the ΔM15 mutation. β-galactosidase can hydrolyze X-Gal, which is 5-bromo-4-chloro-3-indolyl-β-D-galactopyranoside, a colorless compound, to an indigo-colored product and thus allows for a convenient screening method for discriminating between transformants containing starting plasmid pOJ160 and those containing a plasmid pOJ160 derivative, such as plasmid pOJ197.

To prepare E. coli K12 RR1ΔM15 cells that are competent for transformation, the lyophils of E. coli K12 RR1ΔM15 obtained from the NRRL are reconstituted to isolate single colonies. One single-colony isolate of RR1ΔM15 is inoculated into 5 ml of L broth (10 g of Bacto-tryptone, 10 g of NaCl, and 5 g of Bacto-Yeast Extract per liter) that contains 10 mM MgSO₄ and 10 mM MgCl₂, and the culture is incubated at 37°C overnight with aeration. Fifty μl of the overnight culture are used to inoculate 5 ml of L broth that contains 10 mM MgSO₄ and 10 mM MgCl₂. The culture is incubated at 37°C overnight with aeration. The following morning, the culture is diluted to 200 ml with L broth that contains 10 mM MgSO₄ and 10 mM MgCl₂. The diluted culture is incubated at 37°C with aeration until the absorbance at 550 nm ($A_{550}$) is about 0.5, which indicates a cell density of about $1 \times 10^8$ cells/ml. The culture is cooled for ten minutes in an ice-water bath, and the cells are then collected by centrifugation at 4000Xg for 10 minutes at 4°C. The cell pellet is resuspended in 100 ml of cold 10 mM NaCl and then immediately repelleted by centrifugation. The cell pellet is resuspended in 100 ml of 30 mM CaCl₂ and incubated on ice for 20 minutes.

The cells are again collected by centrifugation and resuspended in 10 ml of 30 mM CaCl₂. A one-half ml aliquot of the cells was added to the ligated DNA prepared above. The cell-DNA mixture was incubated on ice for one hour, heat-shocked at 42°C for 90 seconds, and then chilled on ice for about two minutes. The cell-DNA mixture was centrifuged, and the cell pellet was resuspended into 0.5 ml of L broth in a 1.5 ml tube and incubated at 37°C for one hour.

Aliquots of the transformation mixture were plated on L-agar (L-broth with 15 grams per liter agar) plates containing 200 μg apramycin/ml, 40 pg X-gal/ml, and 40 μg IPTG/ml. IPTG serves to derepress the lac promoter present on plasmid pOJ160. The plates were incubated at 37°C overnight. Colonies that contain a plasmid without an insert, such as E. coli K12 RR1ΔM15/pOJ160, appear blue on these plates. Colonies that contain a plasmid with an insert, such as E. coli K12 RR1ΔM15/pOJ197, are white. Several apramycin-resistant, white colonies were selected and then screened by restriction enzyme analysis of their plasmid DNA for the presence of the 4 kb EcoRI-PstI restriction fragment. Plasmid DNA was obtained from the E. coli K12 RR1ΔM15/pOJ197 transformants in accordance with the procedure for isolating plasmid pOJ160 DNA, described above. The plasmid pOJ197 DNA can be used to transform <u>Streptomyces</u> <u>lividans</u> TK23 and <u>Streptomyces</u> <u>griseofuscus</u> C581 to carbomycin resistance, as described in Example 3, below.

<u>Example 3</u>

<u>Construction of Carbomycin-Resistant Streptomyces lividans TK23 and S. grisoefuscus C581</u>

A. <u>List of Solutions</u>

The following solutions are referred to throughout the Examples and are presented here for clarity.

### 1. P medium (∼100 ml):

| Ingredient | Amount |
|---|---|
| Sucrose | 10.3 g |
| $K_2SO_4$ | 0.025 g |
| Trace element solution (see #3) | 0.2 ml |
| $MgCl_2 \cdot 6H_2O$ | 0.203 g |
| Water | 80 ml |

After autoclaving add:

| | |
|---|---|
| $KH_2PO_4$ (0.5%) | 1 ml |
| $CaCl_2 \cdot 2H_2O$ (3.68%) | 10 ml |
| (N-tris-(hydroxymethyl)-methyl-2-aminoethane sulphonic acid), "TES" buffer, 0.25 M, pH=7.2 | 10 ml |

28

2.  Trace element solution (~1 L):

| Ingredient | Amount |
|---|---|
| $ZnCl_2$ | 40 mg |
| $FeCl_3 \cdot 6H_2O$ | 200 mg |
| $CuCl_2 \cdot 2H_2O$ | 10 mg |
| $MnCl_2 \cdot 4H_2O$ | 10 mg |
| $Na_2B_4O_7 \cdot 10H_2O$ | 10 mg |
| $(NH_4)_6Mo_7O_{24} \cdot 4H_2O$ | 10 mg |
| $H_2O$ | 1 L |

3.  R2 Regeneration Medium (~ 1 L):

| Ingredient | Amount |
|---|---|
| Sucrose | 103 g |
| $K_2SO_4$ | 0.25 g |
| Trace element solution | 2 ml |
| $MgCl_2 \cdot 6H_2O$ | 10.12 g |
| glucose | 10 g |
| L-asparagine$\cdot 1H_2O$ | 2.0 g |
| casamino acids | 0.1 g |
| Agar | 22 g |
| Water | to 700 ml |

The pH is adjusted to pH = 7.2 before autoclaving. After autoclaving, add:

| | |
|---|---|
| $KH_2PO_4$ (0.05 g/100 ml) | 100 ml |
| $CaCl_2$ (2.22 g/100 ml) | 100 ml |
| TES Buffer (5.73 g/100 ml, pH = 7.2) | 100 ml |

4.  Soft nutrient agar (SNA, ~1 L):

| Ingredient | Amount |
| --- | --- |
| Difco Bacto Nutrient Broth | 8 g |
| Agar | 5 g |

5.  R2YE medium is R2 medium with 20 ml of 25% yeast extract added per liter.

6.  Yeast Extract - Malt Extract (YEME, ~1 L):

| Ingredient | Amount |
| --- | --- |
| Yeast extract | 3 g |
| Peptone | 5 g |
| Malt extract | 3 g |
| Glucose | 10 g |

7.  YEME + 34% Sucrose Liquid Complete Medium is YEME with 340 g/L of sucrose.

8.  YMX Media (~1 L):

| Ingredient | Amount |
| --- | --- |
| Yeast extract | 3 g |
| Malt extract | 3 g |
| Glucose | 2 g |
| Agar | 20 g |

9.  YMX Agar is 0.3% yeast extract, 0.3% malt extract, 0.2% dextrose, and 2.0% agar.

B. Transformation of Streptomyces griseofuscus

Plasmid pOJ158 was first isolated by transforming a library of MboI-digested Streptomyces thermotolerans (ATCC 11416) DNA cloned into BglII-digested plasmid pIJ702 DNA into S. griseofuscus C581 (ATCC 23916). A number of thiostrepton-resistant transformants were isolated, homogenized, grown in media containing 0.5 μg/ml carbomycin for about 16 hours, and, finally, plated on media containing 10 μg/ml carbomycin. The transforming DNA was prepared from the carbomycinresistant transformants in substantial accordance with the procedure of Example 1. A similar procedure was used with the ligated DNA prepared in Example 2 to obtain S. griseofuscus C581/pOJ197 transformants.

Streptomyces griseofuscus C581 (ATCC 23916) was plated on YMX agar and incubated at 30°C for about 72 hours. A plug of cells was removed from the plate and used to inoculate 10 ml of TSB. The culture was homogenized and incubated at 30°C for 30 hours. About 4 ml of this culture were homogenized and used to inoculate 100 ml of TSB containing 0.4% glycine. The culture was incubated at 30°C for about 24 hours. About 4 ml of this culture were again homogenized and used to inoculate 100 ml of TSB containing 0.4% glycine. The culture was incubated at 30°C for about 16 hours. The cells were harvested and washed three times with 10.3% sucrose. The cells were resuspended in 100 ml of P media containing 1 mg/ml lysozyme, and the resulting solution was incubated at 30°C for 2 hours. During this protoplasting step, the cells were pipetted up and down to disperse clumps. The protoplasts were collected and washed three times with P medium. The protoplasts were then suspended in 10 ml of P medium. This process usually generates about 2 to 5 × 10⁷ protoplasts per 150 μl of solution.

Approximately 150 μl of the protoplast solution were added to 10 μl of the transforming DNA, either in ligation or TE buffer, and mixed. About 101 μl of 50% polyethylene glycol 1000 in P media were then added and mixed. After a brief (1 to 2 minutes) incubation at room temperature, the cell-DNA mixture was brought to a volume of 1 ml by the addition of P media. The cell suspension was plated onto R2 medium; about 0.1 ml of cells was inoculated per R2 plate. The plates were incubated at 30°C overnight (16 hours) and then overlaid with 3 ml of R2-modified agar (103 g sucrose, 10.12 g MgCl₂, 2.22 g CaCl₂, and 5.72 g TES at pH = 7.2 per liter) containing enough thiostrepton to give a final concentration, after diffusion, of 25 μg/ml. The plates were then incubated for about four days at 30°C, when colonies became visible to the naked eye.

Carbomycin-resistant transformants were selected by patching regenerated protoplasts to R2 medium containing 10 μg/ml of carbomycin. Alternatively, carbomycin-resistant transformants were selected by inoculating the regenerated protoplasts into TSB containing 0.5 μg/ml carbomycin. The culture was incubated for 16-22 hours at 30°C before plating the cells onto media containing 10 μg/ml carbomycin. As plasmids pOJ158 and pOJ197 also comprise a thiostrepton resistance-conferring gene, thiostrepton, at a final concentration of 20 μg/ml, was also used to select transformants.

The transformants were cultured on R2 agar supplemented with carbomycin (10 μg/ml) to obtain single colonies. These single colonies were used to inoculate 10 ml TSB cultures containing both carbomycin and thiostrepton (25 μg/ml). The cultures were homogenized and then grown overnight at 30°C in a rotary shaker.

Plasmid isolation for analysis was done by a small-scale version of the protocol of Example 1; the CsCl gradients of Example 1 were replaced by ethanol precipitations. The mycelium was collected by centrifugation, washed twice with 10.3% sucrose and then suspended in 1-2 ml of 10.3% sucrose. Four hundred μl of the cell mixture were transferred to a small tube, and 100 μl of 5X Lysozyme solution (Example 1) were added. The suspension was incubated at 30°C for 30-60 minutes, followed by the addition and mixing of 300 μl of 0.3 M NaOH containing 1% SDS. The latter solution was kept at 50°C before its addition to the cell mix. The cell mixture was placed at 80°C for 10 minutes, cooled to room temperature, and then extracted with 200 μl of phenol:CHCl₃ (50:50). The aqueous phase was transferred to a clean tube, made 0.3 M in NaOAc, and then, one volume of isopropanol was added. The DNA was incubated at room temperature for five minutes and then pelleted by centrifugation. The pellet was dissolved in 500 μl of TE buffer, and about 25 μl of 0.1 M spermine were added to the solution of DNA, and the mixture was incu bated at room temperature for 5 minutes. After centrifugation, the DNA pellet was rinsed with 75% ethanol, then resuspended and reprecipitated from 0.3 M sodium acetate using ethanol. After this last precipitation, the plasmid DNA was suspended in 50 μl of TE buffer. Restriction enzyme cutting and electrophoretic analysis of the reaction products were used to determine plasmid structure.

C. Preparation of Streptomyces lividans Protoplasts and Transformation to Carbomycin Resistance

Streptomyces lividans TK23 (NRRL 15826) was plated on R2 agar, and the plates were incubated at 30°C for 16 hours. A plug of cells was taken from the plate and used to inoculate 10 ml of TSS-glycine (12% sucrose and 0.5% glycine in TSB). This culture was incubated at 30°C for 65 hours with aeration. The culture was then homogenized, sonicated, pelleted with centrifugation, and washed with 10 ml of P media. The cell pellet was resuspended in P media containing 2 mg/ml lysozyme, incubated at 4°C for 15 minutes, mixed by inversion, and then incubated at 4°C for 30 minutes. The resulting protoplasts were washed twice in P media and then resuspended in 10 ml of P media. For each sample of transforming DNA (5 μl), 200 μl of protoplasts were added to the DNA, and then, 0.5 ml of 20% polyethylene glycol 1000 in P media was added to the cell-DNA mixture. The cells were then plated in 200 μl aliquots using 3 ml of R2-modified overlays (103 g sucrose, 10.12 g MgCl₂, 2.22 g CaCl₂, and 5.73 g TES at pH = 7.2 per liter). The plates were incubated at 30°C.

Thiostrepton and carbomycin were added and transformants identified as described for the preparation of Streptomyces griseofuscus transformants. Streptomyces lividans TK23 transformants containing the carA

31

gene were prepared in accordance with the preceding procedure.

## Example 4

Isolation of Plasmid pOJ159

A. Culture of Streptomyces griseofuscus C581/pOJ159

About $10^8$ spores of Streptomyces griseofuscus C581/pOJ159 (NRRL 18090) are inoculated into 10 ml of TSB medium containing 25 µg/ml thiostrepton and grown at 29°C until the culture is in early stationary phase. The culture was then homogenized, and 5 ml of the homogenized culture were used to inoculate 100 ml of TSB also containing thiostrepton. The 100 ml of culture were incubated at 29°C until the Streptomyces griseofuscus C581/pOJ159 cells reached stationary phase.

B. Plasmid Isolation

Plasmid pOJ159 was isolated from the cells prepared in substantial accordance with the procedure of Example 4A as described in Example 1B. The procedure yields about 100 µg of plasmid pOJ159 DNA at a concentration of 0.2 µg/µl, which is stored at 4°C. A restriction site and function map of plasmid pOJ159 is presented in Figure 2 of the accompanying drawings.

## Example 5

Construction of Plasmids pOJ169 and pOJ170

Streptomyces lividans/pIJ702 (ATCC 39155) was cultured and plasmid pIJ702 isolated in substantial accordance with the teaching of Example 1. Thiostrepton selection (25 µg/ml) was used to ensure plasmid pIJ702 maintenance. The 100 µg of plasmid pIJ702 DNA obtained were suspended in 5 ml of TE and stored at 4°C.

About 10 µg (100 µl) of plasmid pIJ702 DNA were added to 13 µl of 10X PstI buffer (500 mM tris-HCl, pH = 7.5; 0.5 M Nacl; and 100 mM MgCl$_2$), 14 µl of H$_2$O, and 3 µl (45 units) of restriction enzyme PstI. The resulting reaction was incubated at 37°C for two hours. The reaction mixture was extracted with 100 µl of a 1:1 solution of phenol:chloroform and then with 100 µl of chloroform. The PstI-digested DNA was collected by adjusting the sodium acetate (NaOAc) concentration of the reaction mixture to 0.30 M, adding two volumes of ethanol, chilling the reaction mixture to -70°C, and centrifuging to pellet the precipitated DNA. The pellet of PstI-digested plasmid pIJ702 DNA was resuspended in 50 µl of TE buffer.

About 10 µg of plasmid pOJ159 in 100 µl of TE buffer were added to 13 µl of 10X PstI buffer, 13 µl of H$_2$O and 4 µl (60 units) of restriction enzyme PstI. The resulting reaction was incubated at 37°C for 2 hours. The PstI-digested plasmid pOJ159 DNA was then electrophoresed on a 1% agarose gel until the desired 3.0 kb PstI restriction fragment was clearly separated from the other digestion products. The 3.0 kb PstI fragment was purified from the gel and other reaction products in accordance with the procedure described in Example 2B. About 2.5 µg of the desired 3.0 kb PstI restriction fragment of plasmid pOJ159 were purified in accordance with the procedure.

About 2 µl (0.2 µg) of the PstI-digested plasmid pIJ702 DNA were added to 10 µl (0.5 µg) of the 3.0 kb, carB-containing PstI restriction fragment of plasmid pOJ159, 2 µl of 10X ligase buffer (660 mM Tris-HCl, pH = 8; 66 mM MgCl$_2$; 10 mM dithiothreitol (DTT); and 10 mM ATP), and 5 µl of H$_2$O. About 1 µl (100 units) of T4 DNA ligase was added to the solution of DNA, and the resulting reaction was incubated at 15°C overnight (16 hours). The ligated DNA constituted the desired plasmids pOJ169 and pOJ170, which differ only with respect to the orientation of the 3.0 kb fragment. A restriction site and function map of plasmid pOJ169 is presented in Figure 4 of the accompanying drawings. The ligated DNA can be used to transform Streptomyces lividans TK23 and Streptomyces griseofuscus C581 as described in Example 6, below.

## Example 6

Construction of Carbomycin-Resistant Streptomyces lividans TK23 and S. grisoefuscus C581

A. Transformation of Streptomyces griseofuscus

Plasmid pOJ159 was first isolated by transforming a library of MboI-digested Streptomyces thermotolerans (ATCC 11416) DNA cloned into BglII-digested plasmid pIJ702 DNA into S. griseofuscus C581 (ATCC 23916). A number of thiostrepton-resistant transformants were isolated, homogenized, grown in media containing 0.5 µg/ml carbomycin for about 16 hours, and, finally, plated on media containing 10 µg/ml carbomycin. The transforming DNA was prepared from the carbomycin-resistant transformants in substantial accordance with the procedure of Example 1. The isolated DNA, which included plasmid pOJ159, was then used to transform S. lividans TK23 (NRRL 15826) to carbomycin resistance. A similar procedure was used with the ligated DNA prepared in Example 5 to obtain S. griseofuscus C581/pOJ169 and S. griseofuscus C581/pOJ170 transformants.

Streptomyces griseofuscus C581 (ATCC 23916) was plated on YMX agar and incubated at 30°C for about 72 hours. A plug of cells was removed from the plate and used to inoculate 10 ml of TSB. The culture was homogenized and incubated at 30°C for 30 hours. About 4 ml of this culture were homogenized and used to inoculate 100 ml of TSB containing 0.4% glycine. The culture was incubated at 30°C for about 24 hours.

About 4 ml of this culture were again homogenized and used to inoculate 100 ml of TSB containing 0.4% glycine. The culture was incubated at 30°C for about 16 hours. The cells were harvested and washed three times with 10.3% sucrose. The cells were resuspended in 100 ml of P media containing 1 mg/ml lysozyme, and the resulting solution was incubated at 30°C for 2 hours. During this protoplasting step, the cells were pipetted up and down to disperse clumps. The protoplasts were collected and washed three times with P medium. The protoplasts were then suspended in 10 ml of P medium. This process usually generates about 2 to 5 × 10^7 protoplasts per 150 μl of solution.

Approximately 150 μl of the protoplast solution were added to 10 μl of the transforming DNA, either in ligation or TE buffer, and mixed. About 101 μl of 50% polyethylene glycol 1000 in P media were then added and mixed. After a brief (1 to 2 minutes) incubation at room temperature, the cell-DNA mixture was brought to a volume of 1 ml by the addition of P media. The cell suspension was plated onto R2 medium; about 0.1 ml of cells was inoculated per R2 plate. The plates were incubated at 30°C overnight (16 hours) and then overlaid with 3 ml of R2-modified agar (103 g sucrose, 10.12 g $MgCl_2$; 2.22 g $CaCl_2$; and 5.72 g TES at pH = 7.2 per liter) containing enough thiostrepton to give a final concentration, after diffusion, of 25 Mg/ml. The plates were then incubated for about four days at 30°C, when colonies became visible to the naked eye.

Carbomycin-resistant transformants were selected by patching regenerated protoplasts to R2 medium containing 10 μg/ml of carbomycin. Alternatively, carbomycin-resistant transformants were selected by inoculating the regenerated protoplasts into TSB containing 0.5 μg/ml carbomycin. The culture was incubated for 16-22 hours at 30°C before plating the cells onto media containing 10 μg/ml carbomycin. As plasmids pOJ159, pOJ169, and pOJ170 also comprise a thiostrepton resistance-conferring gene, thiostrepton, at a final concentration of 20 μg/ml, was also used to select transformants.

The transformants were cultured on R2 agar supplemented with carbomycin (10 μg/ml) to obtain single colonies. These single colonies were used to inoculate 10 ml TSB cultures containing both carbomycin and thiostrepton (25 μg/ml). The cultures were homogenized and then grown overnight at 30°C in a rotary shaker.

Plasmid isolation for analysis was done by a small-scale version of the protocol of Example 1; the CsCl gradients of Example 1 were replaced by ethanol precipitations. The mycelium was collected by centrifugation, washed twice with 10.3% sucrose and then suspended in 1-2 ml of 10.3% sucrose. Four hundred μl of the cell mixture were transferred to a small tube, and 100 μl of 5X Lysozyme solution (Example 1) were added. The suspension was incubated at 30°C for 30-60 minutes, followed by the addition and mixing of 300 μl of 0.3 M NaOH containing 1% SDS. The latter solution was kept at 50°C before its addition to the cell mix. The cell mixture was placed at 80°C for 10 minutes, cooled to room temperature, and then extracted with 200 μl of phenol:$CHCl_3$ (50:50). The aqueous phase was transferred to a clean tube, made 0.3 M in NaOAc, and then, one volume of isopropanol was added. The DNA was incubated at room temperature for five minutes and then pelleted by centrifugation. The pellet was dissolved in 500 μl of TE buffer, and about 25 μl of 0.1 M spermine were added to the solution of DNA, and the mixture was incubated at room temperature for 5 minutes. After centrifugation, the DNA pellet was rinsed with 75% ethanol, then resuspended and reprecipitated from 0.3 M sodium acetate using ethanol. After this last precipitation, the plasmid DNA was suspended in 50 μl of TE buffer. Restriction enzyme cutting and electrophoretic analysis of the reaction products were used to determine plasmid structure.

B. Preparation of Streptomyces lividans Protoplasts and Transformation to Carbomycin Resistance

Streptomyces lividans TK23 (NRRL 15826) was plated on R2 agar, and the plates were incubated at 30°C for 16 hours. A plug of cells was taken from the plate and used to inoculate 10 ml of TSS-glycine (12% sucrose and 0.5% glycine in TSB). This culture was incubated at 30°C for 65 hours with aeration. The culture was then homogenized, sonicated, pelleted with centrifugation, and washed with 10 ml of P media. The cell pellet was resuspended in P media containing 2 mg/ml lysozyme, incubated at 4°C for 15 minutes, mixed by inversion, and then incubated at 4°C for 30 minutes. The resulting protoplasts were washed twice in P media and then resuspended in 10 ml of P media. For each sample of transforming DNA (5 μl), 200 μl of protoplasts were added to the DNA together with 0.5 ml of 20% polyethylene glycol 1000 in P media. The cells were then plated in 200 μl aliquots using 3 ml of R2-modified overlays (103 g sucrose, 10.12 g $MgCl_2$, 2.22 g $CaCl_2$, and 5.73 g TES at pH = 7.2 per liter). The plates were incubated at 30°C.

Thiostrepton and carbomycin were added and transformants identified as described for the preparation of Streptomyces griseofuscus transformants. Streptomyces lividans TK23/pOJ159 transformants were prepared in accordance with the preceding procedure.

**Claims**

1. A method for selecting a recombinant DNA-containing host cell, which comprises:
   (a) transforming a carbomycin-sensitive host cell with a recombinant DNA vector that comprises a DNA sequence that confers resistance to carbomycin; and
   (b) culturing the transformed host cell under growth conditions suitable for the selection of carbomycin-resistant transformants.

2. A DNA sequence that encodes a polypeptide that confers resistance to carbomycin.

3. The DNA sequence of Claim 2 that is the carA gene.

4. The DNA sequence of Claim 2 that is the 4 kb PstI-EcoRI restriction fragment of plasmid pOJ158.

5. A recombinant DNA vector that comprises the DNA sequence of Claim 2.

6. The recombinant DNA vector of Claim 5 that is plasmid pOJ158 or pOJ197.

7. The DNA sequence of Claim 2 that is the carB gene.

8. The DNA sequence of Claim 7 that is:

```
                10        20        30        40
   5'-CGCTGGACTG CCGCCCGGTT TTGAGCAGGC ATTTCGCCCG


                50        60        70        80
      TCCGCCCGCA CGGCGGGGCC AGCCACGGGC CGGTGTCCGC


                90        100       110       120
      CGCCGGCGCC GCGCCACCCC CACGGCCGCA TCCCGTACCC
```

```
            130        140         150        160
GGGCTGCGGC CGCTTCGGGT TGTCCTCTGC CGCGGCTTGA

            170        180         190        200
TTTCACCTCC CCGGGGGGTG TGCGTAGTGG CAGCGAAGGT

            210        220         230        240
AAAGGGAGGT TAAGGTGTCC GTCCGCCGGG GTTCCTGTAC

            250        260         270        280
GTAATGGCTG CGCTCCTGAA GCGCATACTT AGGAGACGCA

            290        300        310         320         330
TGGCTGAAAA GAGGTCAGGA CGCGGGCGC ATG GCC GCA GCG CGT ACA ACC GGA GCT

      340         350        360         370         380
CAG TCG CGT AAA ACG GCA CAG CGG TCG GGC CGG AGT GAG GCT GAC CGT

      390         400        410         420         430
AGA AGA AGA GTC CAC GGG CAG AAT TTC CTC GTC GAC CGG GAA ACA GTA

      440         450        460         470         480
CAA CGG TTT GTG CGT TTC GCC GAT CCG GAC CCC GGG GAG GTC GTT CTC

      490         500        510         520
GAG GTC GGT GCC GGT AAT GGT GCG ATC ACG CGC GAG CTG GCG CGA TTA

530         540        550        560        570
TGC CGA CGA GTG GTG GCG TAT GAG ATC GAC CGG CAC TTC GCG GAC CGA

      580        590        600        610        620
TTA CGT GAG GCG ACC GCC GAG GAT CCG CGG ATC GAG GTC GTC GCC GGC

      630        640        650        660        670
GAC TTC CTG AAG ACC TCG CAG CCC AAG GTC CCG TTC TCC GTG GTC GGC

      680        690        700        710         720
AAC ATC CCG TTC GGC AAC ACC GCG GAC ATA GTG GAC TGG TGC CTG AAC

      730        740        750        760
GCG CGG CGG CTG CGT ACG ACC ACC CTG GTC ACC CAG CTC GAA TAC GCC

770         780        790        800        810
CGC AAG CGC ACC GGC GGC TAT CGG CGC TGG TCA CGG CTC ACC GTG GCC

      820        830        840        850        860
ACC TGG CCC GAG GTG GAG TGG CGG ATG GGC GAG CGG ATC AGC CGC CGC

      870        880        890        900        910
TGG TTC CGG CCC GTC CCC GCC GTC GAC TCC GCG GTA CTG CGA CTG GAA
```

```
      920           930           940           950           960
CGG CGA CCG GTG CCG CTG ATC CCA CCC GGT CTG ATG CAC GAC TTC CGG

            970           980           990          1000
GAC CTG GTG GAG ACC GGG TTC ACG GGA AAG GGC GGT TCG CTG GAC GCC

     1010          1020          1030          1040          1050
TCG CTG CGC CGG CGC TTC CCG GCC CGG CGG GTG GCC GCC GGG TTC CGC

       1060          1070          1080          1090          1100
AGG GCC CGC CTG GAG CAG GGC GTG GTC GTC GCC TAC GTC ACC CCG GGC

         1110          1120          1130          1140          1150
CAA TGG ATC ACA CTC TTC GAG GAA CTC CAC GGG CGC TGA CGCACAC

            1160          1170          1180          1190          1200
  CGGTGCGGGG CGCGCCCGAG GGCGCGCCCC GCACCGGTCG TCCCGCGTCG

            1210          1220          1230          1240          1250
  GCTACGGAGT GCCGGACGGG GCGGGTTCGG ACCTGGGTGT CGAGCCGGCG

            1260
  TCCGGGGACC-3'
```

wherein A is deoxyadenyl, G is deoxyguanyl, C is deoxycytidyl, and T is thymidyl.

9. The DNA sequence of Claim 2 that encodes the carbomycin resistance-conferring gene product that is:

```
                        MET ALA ALA ALA ARG THR THR GLY ALA
                                              5

GLN SER ARG LYS THR ALA GLN ARG SER GLY ARG SER GLU ALA ASP ARG
10                  15                  20                  25

ARG ARG ARG VAL HIS GLY GLN ASN PHE LEU VAL ASP ARG GLU THR VAL
                30                  35                  40

GLN ARG PHE VAL ARG PHE ALA ASP PRO ASP PRO GLY GLU VAL VAL LEU
                45                  50                  55

LEU GLU VAL GLY ALA GLY ASN GLY ALA ILE THR ARG GLU LEU ALA ARG
            60                  65                  70

CYS ARG ARG VAL VAL ALA TYR GLU ILE ASP ARG HIS PHE ALA ASP ARG
        75                  80                  85

LEU ARG GLU ALA THR ALA GLU ASP PRO ARG ILE GLU VAL VAL ALA GLY
90                  95                  100                 105
```

ASP PHE LEU LYS THR SER GLN PRO LYS VAL PRO PHE SER VAL VAL GLY
110 115 120

ASN ILE PRO PHE GLY ASN THR ALA ASP ILE VAL ASP TRP CYS LEU ASN
125 130 135

ALA ARG ARG LEU ARG THR THR THR LEU VAL THR GLN LEU GLU TYR ALA
140 145 150

ARG LYS ARG THR GLY GLY TYR ARG ARG TRP SER ARG LEU THR VAL ALA
155 160 165

THR TRP PRO GLU VAL GLU TRP ARG MET GLY GLU ARG ILE SER ARG ARG
170 175 180 185

TRP PHE ARG PRO VAL PRO ALA VAL ASP SER ALA VAL LEU ARG LEU GLU
190 195 200

ARG ARG PRO VAL PRO LEU ILE PRO PRO GLY LEU MET HIS ASP PHE ARG
205 210 215

ASP LEU VAL GLU THR GLY PHE THR GLY LYS GLY GLY SER LEU ASP ALA
220 225 230

SER LEU ARG ARG ARG PHE PRO ALA ARG ARG VAL ALA ALA GLY PHE ARG
235 240 245

ARG ALA ARG LEU GLU GLN GLY VAL VAL VAL ALA TYR VAL THR PRO GLY
250 255 260 265

GLN TRP ILE THR LEU PHE GLU GLU LEU HIS GLY ARG
270 275

wherein ALA is an alanine, ARG is an arginine, ASN is an asparagine, ASP is an aspartic acid, CYS is a cysteine, GLN is a glutamine, GLU is a glutamic acid, GLY is a glycine, HIS is a histidine, ILE is an isoleucine, LEU is a leucine, LYS is a lysine, MET is a methionine, PHE is a phenylalanine, PRO is a proline, SER is a serine, THR is a threonine, TRP is a tryptophan, TYR is a tyrosine, and VAL is a valine.

10. The DNA sequence of Claim 9 that is:

```
                                      5'-ATG GCC GCA GCG CGT ACA ACC GGA GCT

          CAG TCG CGT AAA ACG GCA CAG CGG TCG GGC CGG AGT GAG GCT GAC CGT

          AGA AGA AGA GTC CAC GGG CAG AAT TTC CTC GTC GAC CGG GAA ACA GTA

          CAA CGG TTT GTG CGT TTC GCC GAT CCG GAC CCC GGG GAG GTC GTT CTC

          GAG GTC GGT GCC GGT AAT GGT GCG ATC ACG CGC GAG CTG GCG CGA TTA

          TGC CGA CGA GTG GTG GCG TAT GAG ATC GAC CGG CAC TTC GCG GAC CGA

          TTA CGT GAG GCG ACC GCC GAG GAT CCG CGG ATC GAG GTC GTC GCC GGC

          GAC TTC CTG AAG ACC TCG CAG CCC AAG GTC CCG TTC TCC GTG GTC GGC

          AAC ATC CCG TTC GGC AAC ACC GCG GAC ATA GTG GAC TGG TGC CTG AAC

          GCG CGG CGG CTG CGT ACG ACC ACC CTG GTC ACC CAG CTC GAA TAC GCC

          CGC AAG CGC ACC GGC GGC TAT CGG CGC TGG TCA CGG CTC ACC GTG GCC

          ACC TGG CCC GAG GTG GAG TGG CGG ATG GGC GAG CGG ATC AGC CGC CGC

          TGG TTC CGG CCC GTC CCC GCC GTC GAC TCC GCG GTA CTG CGA CTG GAA

          CGG CGA CCG GTG CCG CTG ATC CCA CCC GGT CTG ATG CAC GAC TTC CGG

          GAC CTG GTG GAG ACC GGG TTC ACG GGA AAG GGC GGT TCG CTG GAC GCC

          TCG CTG CGC CGG CGC TTC CCG GCC CGG CGG GTG GCC GCC GGG TTC CGC

          AGG GCC CGC CTG GAG CAG GGC GTG GTC GTC GCC TAC GTC ACC CCG GGC

          CAA TGG ATC ACA CTC TTC GAG GAA CTC CAC GGG CGC-3'
```

wherein A is deoxyadenyl, G is deoxyguanyl, C is deoxycytidyl, and T is thymidyl.

11. The recombinant DNA vector of Claim 5 that is plasmid pOJ159, pOJ168, pOJ168.1, pOJ169, or pOJ170.

12. A recombinant host cell transformed with a vector of Claim 5.

13. The recombinant host cell of Claim 12 that is Streptomyces.

14. The recombinant host cell of Claim 13 that is Streptomyces lividans/pOJ159, Streptomyces lividans/pOJ169, Streptomyces lividans/pOJ170, Streptomyces griseofuscus/pOJ159, Streptomyces griseofuscus/pOJ169, Streptomyces griseofuscus/pOJ170, Streptomyces lividans/pOJ158, Streptomyces lividans/pOJ197, Streptomyces griseofuscus/pOJ158 or Streptomyces griseofuscus/pOJ197.

15. The use of the carA or carB gene as a selectable marker.

16. A method for expressing a recombinant gene product that comprises:

 (a) transforming a host cell with a recombinant DNA vector that comprises:

  (i) the promoter of the carA gene positioned for driving expression of

  (ii) a DNA sequence that encodes the recombinant gene product; and

 (b) culturing the transformed host cell under conditions suitable for expression of the gene product.

17. A DNA sequence that comprises the promoter of the carA gene.

18. A recombinant DNA vector that comprises a DNA sequence of Claim 17.

19. A method for expressing a recombinant gene product that comprises:

 a) transforming a host cell with a recombinant DNA vector that comprises:

  (i) a promoter positioned for driving expression of

  (ii) a DNA sequence that encodes the recombinant gene product and

(iii) the terminator of the carA gene positioned to terminate transcription of the recombinant gene product-encoding DNA; and

(b) culturing the transformed host cell under conditions suitable for expression of the gene product.

20. A DNA sequence that comprises the terminator of the carA gene.

21. A method for expressing a recombinant gene product that comprises:

(a) transforming a host cell with a recombinant DNA vector that comprises:

(i) the promoter of the carB gene positioned for driving expression of

(ii) a DNA sequence that encodes the recombinant gene product; and

(b) culturing the transformed host cell under conditions suitable for expression of the gene product.

22. A DNA sequence that comprises the promoter of the carB gene.

23. The DNA sequence of Claim 21 that is:

```
5'-CGCTGGACTG CCGCCCGGTT TTGAGCAGGC ATTTCGCCCG

   TCCGCCCGCA CGGCGGGGCC AGCCACGGGC CGGTGTCCGC

   CGCCGGCGCC GCGCCACCCC CACGGCCGCA TCCCGTACCC

   GGGCTGCGGC CGCTTCGGGT TGTCCTCTGC CGCGGCTTGA

   TTTCACCTCC CCGGGGGGTG TGCGTAGTGG CAGCGAAGGT

   AAAGGGAGGT TAAGGTGTCC GTCCGCCGGG GTTCCTGTAC

   GTAATGGCTG CGCTCCTGAA GCGCATACTT AGGAGACGCA

   TGGCTGAAAA GAGGTCAGGA CGCGGGCGC-3'
```

wherein A is deoxyadenyl, G is deoxyguanyl, C is deoxycytidyl, and T is thymidyl.

24. A method for expressing a recombinant gene product that comprises:

a) transforming a host cell with a recombinant DNA vector that comprises:

(i) a promoter positioned for driving expression of

(ii) a DNA sequence that encodes the recombinant gene product and

(iii) the terminator of the carB gene positioned to terminate transcription of the recombinant gene product-encoding DNA; and

(b) culturing the transformed host cell under conditions suitable for expression of the gene product.

25. A DNA sequence that comprises the terminator of the carB gene.

26. The DNA sequence of Claim 25 that is: 5'-CGCACAC CGGTGCGGGG CGCGCCCGAG GGCGCGCCCC GCACCGGTCG TCCC-3'

wherein A is deoxyadenyl, G is deoxyguanyl, C is deoxycytidyl, and T is thymidyl.

0258046

# FIG.I

### Restriction Site and Function Map of Plasmid pOJ158
### (12.65 kb)

0258046

# FIG.2

## Restriction Site and Function Map of Plasmid pOJ159
### (10.65 kb)

0258046

# FIG.3

Restriction Site and Function Map of
Plasmid pOJ160
(7 kb)

0258046

# FIG.4

### Restriction Site and Function Map of
### Plasmid pOJ169
### (8.72 kb)

0258046

# FIG.5

Restriction Site and Function Map of
Plasmid pOJ197
(9.45 kb)